# EUROPÄISCHE PATENTSCHRIFT

(11) **EP 1 313 768 B1**
(45) Veröffentlichungstag und Bekanntmachung des Hinweises auf die Patenterteilung: **06.02.2008**
(21) Anmeldenummer: 01976176.6
(22) Anmeldetag: 31.08.2001
(51) Int. Cl.: C07K 14/705

(54) **ANTISENSE OLIGONUKLEOTIDE GEGEN VR 1**
ANTISENSE OLIGONUCLEOTIDES AGAINST VR1
OLIGONUCLEOTIDES ANTISENS CONTRE VR1

(30) Priorität: 02.09.2000 DE 10043674; 04.09.2000 DE 10043702
(43) Veröffentlichungstag der Anmeldung: 28.05.2003
(73) Patentinhaber: Grünenthal GmbH, 52078 Aachen (DE)
(72) Erfinder: KURRECK, Jens, 12226 Berlin (DE); ERDMANN, Volker, A., 14163 Berlin (DE)
(86) Internationale Anmeldenummer: PCT/EP2001/010081
(87) Internationale Veröffentlichungsnummer: WO 2002/018407

(56) Entgegenhaltungen:
- WO-A-00/29577
- KURRECK JENS ET AL: "Comparative study of DNA enzymes and ribozymes against the same full-length messenger RNA of the vanilloid receptor subtype I." JOURNAL OF BIOLOGICAL CHEMISTRY, Bd. 277, Nr. 9, 1. März 2002 (2002-03-01), Seiten 7099-7107, XP001068907 March 1, 2002 ISSN: 0021-9258
- CATERINA M J ET AL: "THE CAPSAICIN RECEPTOR: A HEAT-ACTIVATED ION CHANNEL IN THE PAIN PATHWAY" NATURE, MACMILLAN JOURNALS LTD. LONDON, GB, Bd. 389, 23. Oktober 1997 (1997-10-23), Seiten 816-824, XP002942960 ISSN: 0028-0836
- GOILA R ET AL: "Sequence specific cleavage of the HIV-1 coreceptor CCR5 gene by a hammer-head ribozyme and a DNA-enzyme: inhibition of the coreceptor function by DNA-enzyme" FEBS LETTERS, ELSEVIER SCIENCE PUBLISHERS, AMSTERDAM, NL, Bd. 436, Nr. 2, 2. Oktober 1998 (1998-10-02), Seiten 233-238, XP004258427 ISSN: 0014-5793

### SEQUENZ PROTOKOLL

<110> Grünenthal GmbH
<120> Antisense Oligonukleotide gegen VR1
<130> GRA 3027 PCT
<140> PCT / EP 01 / 10081
   <141> 2001-08-31
<150> DE 100 43 674.9
   <151> 2000-09-02
<150> DE 100 43 702.8
   <151> 2000-09-04
<160> 248
<170> PatentIn Ver. 2.1
<210> 1
   <211> 9
   <212> DNA
   <213> Rattus norvegicus
<400> 1
   catgacggt 9
<210> 2
   <211> 15
   <212> DNA
   <213> Rattus norvegicus
<400> 2
   gtcatgacgg ttagg 15
<210> 3
   <211> 15
   <212> DNA
   <213> Rattus norvegicus
<400> 3
   tgtcatgacg gttag 15
<210> 4
   <211> 15
   <212> DNA
   <213> Rattus norvegicus
<400> 4
   atgtcatgac ggtta 15
<210> 5
   <211> 15
   <212> DNA
   <213> Rattus norvegicus
<400> 5
   catgtcatga cggtt 15
<210> 6
   <211> 16
   <212> DNA
   <213> Rattus norvegicus
<400> 6
   tgtcatgacg gttagg 16
<210> 7
   <211> 16
   <212> DNA
   <213> Rattus norvegicus
<400> 7
   atgtcatgac ggttag 16
<210> 8
   <211> 16
   <212> DNA
   <213> Rattus norvegicus
<400> 8
   catgtcatga cggtta 16
<210> 9
   <211> 17
   <212> DNA
   <213> Rattus norvegicus
<400> 9
   atgtcatgac ggttagg 17
<210> 10
   <211> 17
   <212> DNA
   <213> Rattus norvegicus
<400> 10
   catgtcatga cggttag 17
<210> 11
   <211> 18
   <212> DNA
   <213> Rattus norvegicus
<400> 11
   catgtcatga cggttagg 18
<210> 12
   <211> 15
   <212> DNA
   <213> Rattus norvegicus
<400> 12
   gtcatgangg ttagg 15
<210> 13
   <211> 17
   <212> DNA
   <213> Rattus norvegicus
<400> 13
   tgtcatgang gttaggg 17
<210> 14
   <211> 19
   <212> DNA
   <213> Rattus norvegicus
<400> 14
   atgtcatgan ggttagggg 19
<210> 15
   <211> 15
   <212> RNA
   <213> Rattus norvegicus
<400> 15
   augucaunac gguua 15
<210> 16
   <211> 17
   <212> RNA
   <213> Rattus norvegicus
<400> 16
   caugucauna cgguuag 17
<210> 17
   <211> 19
   <212> RNA
   <213> Rattus norvegicus
<400> 17
   gcaugucaun acgguuagg 19
<210> 18
   <211> 9
   <212> DNA
   <213> Rattus norvegicus
<400> 18
   cgtggcgat 9
<210> 19
   <211> 15
   <212> DNA
   <213> Rattus norvegicus
<400> 19
   gtcgtggcga ttagg 15
<210> 20
   <211> 15
   <212> DNA
   <213> Rattus norvegicus
<400> 20
   tgtcgtggcg attag 15
<210> 21
   <211> 15
   <212> DNA
   <213> Rattus norvegicus
<400> 21
   atgtcgtggc gatta 15
<210> 22
   <211> 15
   <212> DNA
   <213> Rattus norvegicus
<400> 22
   catgtcgtgg cgatt 15
<210> 23
   <211> 16
   <212> DNA
   <213> Rattus norvegicus
<400> 23
   tgtcgtggcg attagg 16
<210> 24
   <211> 16
   <212> DNA
   <213> Rattus norvegicus
<400> 24
   atgtcgtggc gattag 16
<210> 25
   <211> 16
   <212> DNA
   <213> Rattus norvegicus
<400> 25
   catgtcgtgg cgatta 16
<210> 26
   <211> 17
   <212> DNA
   <213> Rattus norvegicus
<400> 26
   atgtcgtggc gattagg 17
<210> 27
   <211> 17
   <212> DNA
   <213> Rattus norvegicus
<400> 27
   catgtcgtgg cgattag 17
<210> 28
   <211> 18
   <212> DNA
   <213> Rattus norvegicus
<400> 28
   catgtcgtgg cgattagg 18
<210> 29
   <211> 9
   <212> DNA
   <213> Rattus norvegicus
<400> 29
   gttgacggt 9
<210> 30
   <211> 15
   <212> DNA
   <213> Rattus norvegicus
<400> 30
   ttgttgacgg tctca 15
<210> 31
   <211> 15
   <212> DNA
   <213> Rattus norvegicus
<400> 31
   cttgttgacg gtctc 15
<210> 32
   <211> 15
   <212> DNA
   <213> Rattus norvegicus
<400> 32
   tcttgttgac ggtct 15
<210> 33
   <211> 15
   <212> DNA
   <213> Rattus norvegicus
<400> 33
   atcttgttga cggtc 15
<210> 34
   <211> 16
   <212> DNA
   <213> Rattus norvegicus
<400> 34
   cttgttgacg gtctca 16
<210> 35
   <211> 16
   <212> DNA
   <213> Rattus norvegicus
<400> 35
   tcttgttgac ggtctc 16
<210> 36
   <211> 16
   <212> DNA
   <213> Rattus norvegicus
<400> 36
   atcttgttga cggtct 16
<210> 37
   <211> 17
   <212> DNA
   <213> Rattus norvegicus
<400> 37
   tcttgttgac ggtctca 17
<210> 38
   <211> 17
   <212> DNA
   <213> Rattus norvegicus
<400> 38
   atcttgttga cggtctc 17
<210> 39
   <211> 18
   <212> DNA
   <213> Rattus norvegicus
<400> 39
   atcttgttga cggtctca 18
<210> 40
   <211> 15
   <212> DNA
   <213> Rattus norvegicus
<400> 40
   ttgttgangg tctca 15
<210> 41
   <211> 17
   <212> DNA
   <213> Rattus norvegicus
<400> 41
   cttgttgang gtctcac 17
<210> 42
   <211> 19
   <212> DNA
   <213> Rattus norvegicus
<400> 42
   tcttgttgan ggtctcacc 19
<210> 43
   <211> 15
   <212> RNA
   <213> Rattus norvegicus
<400> 43
   ucuuguunac ggucu 15
<210> 44
   <211> 17
   <212> RNA
   <213> Rattus norvegicus
<400> 44
   aucuuguuna cggucuc 17
<210> 45
   <211> 19
   <212> RNA
   <213> Rattus norvegicus
<400> 45
   aaucuuguun acggucuca 19
<210> 46
   <211> 9
   <212> DNA
   <213> Rattus norvegicus
<400> 46
   gttgacagt 9
<210> 47
   <211> 15
   <212> DNA
   <213> Rattus norvegicus
<400> 47
   ttgttgacag tctca 15
<210> 48
   <211> 15
   <212> DNA
   <213> Rattus norvegicus
<400> 48
   cttgttgaca gtctc 15
<210> 49
   <211> 15
   <212> DNA
   <213> Rattus norvegicus
<400> 49
   tcttgttgac agtct 15
<210> 50
   <211> 15
   <212> DNA
   <213> Rattus norvegicus
<400> 50
   atcttgttga cagtc 15
<210> 51
   <211> 16
   <212> DNA
   <213> Rattus norvegicus
<400> 51
   cttgttgaca gtctca 16
<210> 52
   <211> 16
   <212> DNA
   <213> Rattus norvegicus
<400> 52
   tcttgttgac agtctc 16
<210> 53
   <211> 16
   <212> DNA
   <213> Rattus norvegicus
<400> 53
   atcttgttga cagtct 16
<210> 54
   <211> 17
   <212> DNA
   <213> Rattus norvegicus
<400> 54
   tcttgttgac agtctca 17
<210> 55
   <211> 17
   <212> DNA
   <213> Rattus norvegicus
<400> 55
   atcttgttga cagtctc 17
<210> 56
   <211> 18
   <212> DNA
   <213> Rattus norvegicus
<400> 56
   atcttgttga cagtctca 18
<210> 57
   <211> 15
   <212> DNA
   <213> Rattus norvegicus
<400> 57
   ttgttganag tctca 15
<210> 58
   <211> 17
   <212> DNA
   <213> Rattus norvegicus
<400> 58
   cttgttgana gtctcan 17
<210> 59
   <211> 19
   <212> DNA
   <213> Rattus norvegicus
<400> 59
   tcttgttgan agtctcann 19
<210> 60
   <211> 15
   <212> RNA
   <213> Rattus norvegicus
<400> 60
   ucuuguunac agucu 15
<210> 61
   <211> 17
   <212> RNA
   <213> Rattus norvegicus
<400> 61
   aucuuguuna cagucuc 17
<210> 62
   <211> 19
   <212> RNA
   <213> Rattus norvegicus
<400> 62
   naucuuguun acagucuca 19
<210> 63
   <211> 9
   <212> DNA
   <213> Rattus norvegicus
<400> 63
   cctgacctc 9
<210> 64
   <211> 15
   <212> DNA
   <213> Rattus norvegicus
<400> 64
   ggcctgacct caggg 15
<210> 65
   <211> 15
   <212> DNA
   <213> Rattus norvegicus
<400> 65
   cggcctgacc tcagg 15
<210> 66
   <211> 15
   <212> DNA
   <213> Rattus norvegicus
<400> 66
   tcggcctgac ctcag 15
<210> 67
   <211> 15
   <212> DNA
   <213> Rattus norvegicus
<400> 67
   ctcggcctga cctca 15
<210> 68
   <211> 16
   <212> DNA
   <213> Rattus norvegicus
<400> 68
   cggcctgacc tcaggg 16
<210> 69
   <211> 16
   <212> DNA
   <213> Rattus norvegicus
<400> 69
   tcggcctgac ctcagg 16
<210> 70
   <211> 16
   <212> DNA
   <213> Rattus norvegicus
<400> 70
   ctcggcctga cctcag 16
<210> 71
   <211> 17
   <212> DNA
   <213> Rattus norvegicus
<400> 71
   tcggcctgac ctcaggg 17
<210> 72
   <211> 17
   <212> DNA
   <213> Rattus norvegicus
<400> 72
   ctcggcctga cctcagg 17
<210> 73
   <211> 18
   <212> DNA
   <213> Rattus norvegicus
<400> 73
   ctcggcctga cctcaggg 18
<210> 74
   <211> 15
   <212> DNA
   <213> Rattus norvegicus
<400> 74
   ggcctganct caggg 15
<210> 75
   <211> 17
   <212> DNA
   <213> Rattus norvegicus
<400> 75
   cggcctganc tcaggga 17
<210> 76
   <211> 19
   <212> DNA
   <213> Rattus norvegicus
<400> 76
   tcggcctgan ctcagggag 19
<210> 77
   <211> 15
   <212> RNA
   <213> Rattus norvegicus
<400> 77
   ucggccunac cucag 15
<210> 78
   <211> 17
   <212> RNA
   <213> Rattus norvegicus
<400> 78
   cucggccuna ccucagg 17
<210> 79
   <211> 19
   <212> RNA
   <213> Rattus norvegicus
<400> 79
   ncucggccun accucaggg 19
<210> 80
   <211> 9
   <212> DNA
   <213> Rattus norvegicus
<400> 80
   cttgaccgc 9
<210> 81
   <211> 15
   <212> DNA
   <213> Rattus norvegicus
<400> 81
   tgcttgaccg caggg 15
<210> 82
   <211> 15
   <212> DNA
   <213> Rattus norvegicus
<400> 82
   ctgcttgacc gcagg 15
<210> 83
   <211> 15
   <212> DNA
   <213> Rattus norvegicus
<400> 83
   tctgcttgac cgcag 15
<210> 84
   <211> 15
   <212> DNA
   <213> Rattus norvegicus
<400> 84
   ctctgcttga ccgca 15
<210> 85
   <211> 16
   <212> DNA
   <213> Rattus norvegicus
<400> 85
   ctgcttgacc gcaggg 16
<210> 86
   <211> 16
   <212> DNA
   <213> Rattus norvegicus
<400> 86
   tctgcttgac cgcagg 16
<210> 87
   <211> 16
   <212> DNA
   <213> Rattus norvegicus
<400> 87
   ctctgcttga ccgcag 16
<210> 88
   <211> 17
   <212> DNA
   <213> Rattus norvegicus
<400> 88
   tctgcttgac cgcaggg 17
<210> 89
   <211> 17
   <212> DNA
   <213> Rattus norvegicus
<400> 89
   ctctgcttga ccgcagg 17
<210> 90
   <211> 18
   <212> DNA
   <213> Rattus norvegicus
<400> 90
   ctctgcttga ccgcaggg 18
<210> 91
   <211> 15
   <212> DNA
   <213> Rattus norvegicus
<400> 91
   tgcttgancg caggg 15
<210> 92
   <211> 17
   <212> DNA
   <213> Rattus norvegicus
<400> 92
   ctgcttganc gcagggn 17
<210> 93
   <211> 19
   <212> DNA
   <213> Rattus norvegicus
<400> 93
   tctgcttgan cgcagggnn 19
<210> 94
   <211> 15
   <212> RNA
   <213> Rattus norvegicus
<400> 94
   ucugcuunac cgcag 15
<210> 95
   <211> 17
   <212> RNA
   <213> Rattus norvegicus
<400> 95
   cucugcuuna ccgcagg 17
<210> 96
   <211> 19
   <212> RNA
   <213> Rattus norvegicus
<400> 96
   ncucugcuun accgcaggg 19
<210> 97
   <211> 9
   <212> DNA
   <213> Rattus norvegicus
<400> 97
   gtggactcc 9
<210> 98
   <211> 15
   <212> DNA
   <213> Rattus norvegicus
<400> 98
   gtgtggactc catag 15
<210> 99
   <211> 15
   <212> DNA
   <213> Rattus norvegicus
<400> 99
   ggtgtggact ccata 15
<210> 100
   <211> 15
   <212> DNA
   <213> Rattus norvegicus
<400> 100
   tggtgtggac tccat 15
<210> 101
   <211> 15
   <212> DNA
   <213> Rattus norvegicus
<400> 101
   gtggtgtgga ctcca 15
<210> 102
   <211> 16
   <212> DNA
   <213> Rattus norvegicus
<400> 102
   ggtgtggact ccatag 16
<210> 103
   <211> 16
   <212> DNA
   <213> Rattus norvegicus
<400> 103
   tggtgtggac tccata 16
<210> 104
   <211> 16
   <212> DNA
   <213> Rattus norvegicus
<400> 104
   gtggtgtgga ctccat 16
<210> 105
   <211> 17
   <212> DNA
   <213> Rattus norvegicus

<400> 105
   tggtgtggac tccatag 17
<210> 106
   <211> 17
   <212> DNA
   <213> Rattus norvegicus
<400> 106
   gtggtgtgga ctccata 17
<210> 107
   <211> 18
   <212> DNA
   <213> Rattus norvegicus
<400> 107
   gtggtgtgga ctccatag 18
<210> 108
   <211> 15
   <212> DNA
   <213> Rattus norvegicus
<400> 108
   gtgtggantc catag 15
<210> 109
   <211> 17
   <212> DNA
   <213> Rattus norvegicus
<400> 109
   ggtgtggant ccatagg 17
<210> 110
   <211> 19
   <212> DNA
   <213> Rattus norvegicus
<400> 110
   tggtgtggan tccataggc 19
<210> 111
   <211> 15
   <212> RNA
   <213> Rattus norvegicus
<400> 111
   uggugugnac uccau 15
<210> 112
   <211> 17
   <212> RNA
   <213> Rattus norvegicus
<400> 112
   guggugugna cuccaua 17
<210> 113
   <211> 19
   <212> RNA
   <213> Rattus norvegicus
<400> 113
   nguggugugn acuccauag 19
<210> 114
   <211> 9
   <212> DNA
   <213> Rattus norvegicus
<400> 114
   gtggactca 9
<210> 115
   <211> 15
   <212> DNA
   <213> Rattus norvegicus
<400> 115
   acgtggactc agacg 15
<210> 116
   <211> 15
   <212> DNA
   <213> Rattus norvegicus
<400> 116
   gacgtggact cagac 15
<210> 117
   <211> 15
   <212> DNA
   <213> Rattus norvegicus
<400> 117
   cgacgtggac tcaga 15
<210> 118
   <211> 15
   <212> DNA
   <213> Rattus norvegicus
<400> 118
   gcgacgtgga ctcag 15
<210> 119
   <211> 16
   <212> DNA
   <213> Rattus norvegicus
<400> 119
   gacgtggact cagacg 16
<210> 120
   <211> 16
   <212> DNA
   <213> Rattus norvegicus
<400> 120
   cgacgtggac tcagac 16
<210> 121
   <211> 16
   <212> DNA
   <213> Rattus norvegicus
<400> 121
   gcgacgtgga ctcaga 16
<210> 122
   <211> 17
   <212> DNA
   <213> Rattus norvegicus
<400> 122
   cgacgtggac tcagacg 17
<210> 123
   <211> 17
   <212> DNA
   <213> Rattus norvegicus
<400> 123
   gcgacgtgga ctcagac 17
<210> 124
   <211> 18
   <212> DNA
   <213> Rattus norvegicus
<400> 124
   gcgacgtgga ctcagacg 18
<210> 125
   <211> 15
   <212> DNA
   <213> Rattus norvegicus
<400> 125
   acgtggantc agacg 15
<210> 126
   <211> 17
   <212> DNA
   <213> Rattus norvegicus
<400> 126
   gacgtggant cagacgn 17
<210> 127
   <211> 19
   <212> DNA
   <213> Rattus norvegicus
<400> 127
   cgacgtggan tcagacgnn 19
<210> 128
   <211> 15
   <212> RNA
   <213> Rattus norvegicus
<400> 128
   cgacgugnac ucaga 15
<210> 129
   <211> 17
   <212> RNA
   <213> Rattus norvegicus
<400> 129
   gcgacgugna cucagac 17
<210> 130
   <211> 19
   <212> RNA
   <213> Rattus norvegicus
<400> 130
   ngcgacgugn acucagacg 19
<210> 131
   <211> 9
   <212> DNA
   <213> Rattus norvegicus
<400> 131
   ggggactca 9
<210> 132
   <211> 15
   <212> DNA
   <213> Rattus norvegicus
<400> 132
   gtggggactc agact 15
<210> 133
   <211> 15
   <212> DNA
   <213> Rattus norvegicus
<400> 133
   ggtggggact cagac 15
<210> 134
   <211> 15
   <212> DNA
   <213> Rattus norvegicus
<400> 134
   gggtggggac tcaga 15
<210> 135
   <211> 15
   <212> DNA
   <213> Rattus norvegicus
<400> 135
   ggggtgggga ctcag 15
<210> 136
   <211> 16
   <212> DNA
   <213> Rattus norvegicus
<400> 136
   ggtggggact cagact 16
<210> 137
   <211> 16
   <212> DNA
   <213> Rattus norvegicus
<400> 137
   gggtggggac tcagac 16
<210> 138
   <211> 16
   <212> DNA
   <213> Rattus norvegicus
<400> 138
   ggggtgggga ctcaga 16
<210> 139
   <211> 17
   <212> DNA
   <213> Rattus norvegicus
<400> 139
   gggtggggac tcagact 17
<210> 140
   <211> 17
   <212> DNA
   <213> Rattus norvegicus
<400> 140
   ggggtgggga ctcagac 17
<210> 141
   <211> 18
   <212> DNA
   <213> Rattus norvegicus
<400> 141
   ggggtgggga ctcagact 18
<210> 142
   <211> 15
   <212> DNA
   <213> Rattus norvegicus
<400> 142
   gtggggantc agact 15
<210> 143
   <211> 17
   <212> DNA
   <213> Rattus norvegicus
<400> 143
   ggtggggant cagactc 17
<210> 144
   <211> 19
   <212> DNA
   <213> Rattus norvegicus
<400> 144
   gggtggggan tcagactcc 19
<210> 145
   <211> 15
   <212> RNA
   <213> Rattus norvegicus
<400> 145
   gggugggnac ucaga 15
<210> 146
   <211> 17
   <212> RNA
   <213> Rattus norvegicus
<400> 146
   ggggugggna cucagac 17
<210> 147
   <211> 19
   <212> RNA
   <213> Rattus norvegicus
<400> 147
   nggggugggn acucagacu 19
<210> 148
   <211> 9
   <212> DNA
   <213> Rattus norvegicus
<400> 148
   gggtccgca 9
<210> 149
   <211> 15
   <212> DNA
   <213> Rattus norvegicus
<400> 149
   gtgggtccgc agcag 15
<210> 150
   <211> 15
   <212> DNA
   <213> Rattus norvegicus
<400> 150
   agtgggtccg cagca 15
<210> 151
   <211> 15
   <212> DNA
   <213> Rattus norvegicus
<400> 151
   gagtgggtcc gcagc 15
<210> 152
   <211> 15
   <212> DNA
   <213> Rattus norvegicus
<400> 152
   ggagtgggtc cgcag 15
<210> 153
   <211> 16
   <212> DNA
   <213> Rattus norvegicus
<400> 153
   agtgggtccg cagcag 16
<210> 154
   <211> 16
   <212> DNA
   <213> Rattus norvegicus
<400> 154
   gagtgggtcc gcagca 16
<210> 155
   <211> 16
   <212> DNA
   <213> Rattus norvegicus
<400> 155
   ggagtgggtc cgcagc 16
<210> 156
   <211> 17
   <212> DNA
   <213> Rattus norvegicus
<400> 156
   gagtgggtcc gcagcag 17
<210> 157
   <211> 17
   <212> DNA
   <213> Rattus norvegicus
<400> 157
   ggagtgggtc cgcagca 17
<210> 158
   <211> 18
   <212> DNA
   <213> Rattus norvegicus
<400> 158
   ggagtgggtc cgcagcag 18
<210> 159
   <211> 9
   <212> DNA
   <213> Rattus norvegicus
<400> 159
   cttgacaaa 9
<210> 160
   <211> 15
   <212> DNA
   <213> Rattus norvegicus
<400> 160
   cgcttgacaa atctg 15
<210> 161
   <211> 15
   <212> DNA
   <213> Rattus norvegicus
<400> 161
   gcgcttgaca aatct 15
<210> 162
   <211> 15
   <212> DNA
   <213> Rattus norvegicus
<400> 162
   tgcgcttgac aaatc 15
<210> 163
   <211> 15
   <212> DNA
   <213> Rattus norvegicus
<400> 163
   atgcgcttga caaat 15
<210> 164
   <211> 16
   <212> DNA
   <213> Rattus norvegicus
<400> 164
   gcgcttgaca aatctg 16
<210> 165
   <211> 16
   <212> DNA
   <213> Rattus norvegicus
<400> 165
   tgcgcttgac aaatct 16
<210> 166
   <211> 16
   <212> DNA
   <213> Rattus norvegicus
<400> 166
   atgcgcttga caaatc 16
<210> 167
   <211> 17
   <212> DNA
   <213> Rattus norvegicus
<400> 167
   tgcgcttgac aaatctg 17
<210> 168
   <211> 17
   <212> DNA
   <213> Rattus norvegicus
<400> 168
   atgcgcttga caaatct 17
<210> 169
   <211> 18
   <212> DNA
   <213> Rattus norvegicus
<400> 169
   atgcgcttga caaatctg 18
<210> 170
   <211> 15
   <212> DNA
   <213> Rattus norvegicus
<400> 170
   cgcttganaa atctg 15
<210> 171
   <211> 17
   <212> DNA
   <213> Rattus norvegicus
<400> 171
   gcgcttgana aatctgt 17
<210> 172
   <211> 19
   <212> DNA
   <213> Rattus norvegicus
<400> 172
   tgcgcttgan aaatctgtc 19
<210> 173
   <211> 15
   <212> RNA
   <213> Rattus norvegicus
<400> 173
   ugcgcuunac aaauc 15
<210> 174
   <211> 17
   <212> RNA
   <213> Rattus norvegicus
<400> 174
   augcgcuuna caaaucu 17
<210> 175
   <211> 19
   <212> RNA
   <213> Rattus norvegicus
<400> 175
   gaugcgcuun acaaaucug 19
<210> 176
   <211> 9
   <212> DNA
   <213> Rattus norvegicus
<400> 176
   cttgacgaa 9
<210> 177
   <211> 15
   <212> DNA
   <213> Rattus norvegicus
<400> 177
   cgcttgacga atctg 15
<210> 178
   <211> 15
   <212> DNA
   <213> Rattus norvegicus
<400> 178
   gcgcttgacg aatct 15
<210> 179
   <211> 15
   <212> DNA
   <213> Rattus norvegicus
<400> 179
   tgcgcttgac gaatc 15
<210> 180
   <211> 15
   <212> DNA
   <213> Rattus norvegicus
<400> 180
   atgcgcttga cgaat 15
<210> 181
   <211> 16
   <212> DNA
   <213> Rattus norvegicus
<400> 181
   gcgcttgacg aatctg 16
<210> 182
   <211> 16
   <212> DNA
   <213> Rattus norvegicus
<400> 182
   tgcgcttgac gaatct 16
<210> 183
   <211> 16
   <212> DNA
   <213> Rattus norvegicus
<400> 183
   atgcgcttga cgaatc 16
<210> 184
   <211> 17
   <212> DNA
   <213> Rattus norvegicus
<400> 184
   tgcgcttgac gaatctg 17
<210> 185
   <211> 17
   <212> DNA
   <213> Rattus norvegicus
<400> 185
   atgcgcttga cgaatct 17
<210> 186
   <211> 18
   <212> DNA
   <213> Rattus norvegicus
<400> 186
   atgcgcttga cgaatctg 18
<210> 187
   <211> 15
   <212> DNA
   <213> Rattus norvegicus
<400> 187
   cgcttganga atctg 15
<210> 188
   <211> 17
   <212> DNA
   <213> Rattus norvegicus
<400> 188
   gcgcttgang aatctgn 17
<210> 189
   <211> 19
   <212> DNA
   <213> Rattus norvegicus
<400> 189
   tgcgcttgan gaatctgnn 19
<210> 190
   <211> 15
   <212> RNA
   <213> Rattus norvegicus
<400> 190
   ugcgcuunac gaauc 15
<210> 191
   <211> 17
   <212> RNA
   <213> Rattus norvegicus
<400> 191
   augcgcuuna cgaaucu 17
<210> 192
   <211> 19
   <212> RNA
   <213> Rattus norvegicus
<400> 192
   naugcgcuun acgaaucug 19
<210> 193
   <211> 9
   <212> DNA
   <213> Rattus norvegicus
<400> 193
   ccagacatg 9
<210> 194
   <211> 15
   <212> DNA
   <213> Rattus norvegicus
<400> 194
   ctccagacat gtgga 15
<210> 195
   <211> 15
   <212> DNA
   <213> Rattus norvegicus
<400> 195
   gctccagaca tgtgg 15
<210> 196
   <211> 15
   <212> DNA
   <213> Rattus norvegicus
<400> 196
   agctccagac atgtg 15
<210> 197
   <211> 15
   <212> DNA
   <213> Rattus norvegicus
<400> 197
   cagctccaga catgt 15
<210> 198
   <211> 16
   <212> DNA
   <213> Rattus norvegicus
<400> 198
   gctccagaca tgtgga 16
<210> 199
   <211> 16
   <212> DNA
   <213> Rattus norvegicus
<400> 199
   agctccagac atgtgg 16
<210> 200
   <211> 16
   <212> DNA
   <213> Rattus norvegicus
<400> 200
   cagctccaga catgtg 16
<210> 201
   <211> 17
   <212> DNA
   <213> Rattus norvegicus
<400> 201
   agctccagac atgtgga 17
<210> 202
   <211> 17
   <212> DNA
   <213> Rattus norvegicus
<400> 202
   cagctccaga catgtgg 17
<210> 203
   <211> 18
   <212> DNA
   <213> Rattus norvegicus
<400> 203
   cagctccaga catgtgga 18
<210> 204
   <211> 15
   <212> DNA
   <213> Rattus norvegicus
<400> 204
   ctccaganat gtgga 15
<210> 205
   <211> 17
   <212> DNA
   <213> Rattus norvegicus
<400> 205
   gctccagana tgtggaa 17
<210> 206
   <211> 19
   <212> DNA
   <213> Rattus norvegicus
<400> 206
   agctccagan atgtggaat 19
<210> 207
   <211> 15
   <212> RNA
   <213> Rattus norvegicus
<400> 207
   agcuccanac augug 15
<210> 208
   <211> 17
   <212> RNA
   <213> Rattus norvegicus
<400> 208
   cagcuccana caugugg 17
<210> 209
   <211> 19
   <212> RNA
   <213> Rattus norvegicus
<400> 209
   ncagcuccan acaugugga 19
<210> 210
   <211> 9
   <212> DNA
   <213> Rattus norvegicus
<400> 210
   ccaggcagg 9
<210> 211
   <211> 15
   <212> DNA
   <213> Rattus norvegicus
<400> 211
   ctccaggcag gtgga 15
<210> 212
   <211> 15
   <212> DNA
   <213> Rattus norvegicus

<400> 212
   gctccaggca ggtgg 15
<210> 213
   <211> 15
   <212> DNA
   <213> Rattus norvegicus
<400> 213
   agctccaggc aggtg 15
<210> 214
   <211> 15
   <212> DNA
   <213> Rattus norvegicus
<400> 214
   cagctccagg caggt 15
<210> 215
   <211> 16
   <212> DNA
   <213> Rattus norvegicus
<400> 215
   gctccaggca ggtgga 16
<210> 216
   <211> 16
   <212> DNA
   <213> Rattus norvegicus
<400> 216
   agctccaggc aggtgg 16
<210> 217
   <211> 16
   <212> DNA
   <213> Rattus norvegicus
<400> 217
   cagctccagg caggtg 16
<210> 218
   <211> 17
   <212> DNA
   <213> Rattus norvegicus
<400> 218
   agctccaggc aggtgga 17
<210> 219
   <211> 17
   <212> DNA
   <213> Rattus norvegicus
<400> 219
   cagctccagg caggtgg 17
<210> 220
   <211> 18
   <212> DNA
   <213> Rattus norvegicus
<400> 220
   cagctccagg caggtgga 18
<210> 221
   <211> 9
   <212> DNA
   <213> Rattus norvegicus
<400> 221
   tacgactcc 9
<210> 222
   <211> 15
   <212> DNA
   <213> Rattus norvegicus
<400> 222
   ggtacgactc ctggt 15
<210> 223
   <211> 15
   <212> DNA
   <213> Rattus norvegicus
<400> 223
   gggtacgact cctgg 15
<210> 224
   <211> 15
   <212> DNA
   <213> Rattus norvegicus
<400> 224
   cgggtacgac tcctg 15
<210> 225
   <211> 15
   <212> DNA
   <213> Rattus norvegicus
<400> 225
   ccgggtacga ctcct 15
<210> 226
   <211> 16
   <212> DNA
   <213> Rattus norvegicus
<400> 226
   gggtacgact cctggt 16
<210> 227
   <211> 16
   <212> DNA
   <213> Rattus norvegicus
<400> 227
   cgggtacgac tcctgg 16
<210> 228
   <211> 16
   <212> DNA
   <213> Rattus norvegicus
<400> 228
   ccgggtacga ctcctg 16
<210> 229
   <211> 17
   <212> DNA
   <213> Rattus norvegicus
<400> 229
   cgggtacgac tcctggt 17
<210> 230
   <211> 17
   <212> DNA
   <213> Rattus norvegicus
<400> 230
   ccgggtacga ctcctgg 17
<210> 231
   <211> 18
   <212> DNA
   <213> Rattus norvegicus
<400> 231
   ccgggtacga ctcctggt 18
<210> 232
   <211> 15
   <212> DNA
   <213> Rattus norvegicus
<400> 232
   ggtacgantc ctggt 15
<210> 233
   <211> 17
   <212> DNA
   <213> Rattus norvegicus
<400> 233
   gggtacgant cctggta 17
<210> 234
   <211> 19
   <212> DNA
   <213> Rattus norvegicus
<400> 234
   cgggtacgan tcctggtag 19
<210> 235
   <211> 15
   <212> RNA
   <213> Rattus norvegicus
<400> 235
   cggguacnac uccug 15
<210> 236.
   <211> 17
   <212> RNA
   <213> Rattus norvegicus
<400> 236
   ccggguacna cuccugg 17
<210> 237
   <211> 19
   <212> RNA
   <213> Rattus norvegicus
<400> 237
   nccggguacn acuccuggu 19
<210> 238
   <211> 9
   <212> DNA
   <213> Rattus norvegicus
<400> 238
   tgcggctct 9
<210> 239
   <211> 15
   <212> DNA
   <213> Rattus norvegicus
<400> 239
   ggtgcggctc ttggc 15
<210> 240
   <211> 15
   <212> DNA
   <213> Rattus norvegicus
<400> 240
   gggtgcggct cttgg 15
<210> 241
   <211> 15
   <212> DNA
   <213> Rattus norvegicus
<400> 241
   cgggtgcggc tcttg 15
<210> 242
   <211> 15
   <212> DNA
   <213> Rattus norvegicus
<400> 242
   ccgggtgcgg ctctt 15
<210> 243
   <211> 16
   <212> DNA
   <213> Rattus norvegicus
<400> 243
   gggtgcggct cttggc 16
<210> 244
   <211> 16
   <212> DNA
   <213> Rattus norvegicus
<400> 244
   cgggtgcggc tcttgg 16
<210> 245
   <211> 16
   <212> DNA
   <213> Rattus norvegicus
<400> 245
   ccgggtgcgg ctcttg 16
<210> 246
   <211> 17
   <212> DNA
   <213> Rattus norvegicus
<400> 246
   cgggtgcggc tcttggc 17
<210> 247
   <211> 17
   <212> DNA
   <213> Rattus norvegicus
<400> 247
   ccgggtgcgg ctcttgg 17
<210> 248
   <211> 18
   <212> DNA
   <213> Rattus norvegicus
<400> 248
   ccgggtgcgg ctcttggc 18

## Beschreibung

Die Erfindung betrifft Antisense Oligodesoxynucleotide gegen VR1, entsprechende Nukleotid-Konstrukte, diese enthaltende Zellen, Arzneimittel und Diagnostika, deren Verwendung in der Schmerztherapie und Verfahren zur Diagnose mit VR1 verbundener Symptome und zur Identifizierung schmerzmodulierender Substanzen.

Die effektive Behandlung von Schmerz ist eine große Herausforderung für die molekulare Medizin. Akuter und transitorischer Schmerz ist ein wichtiges Signal des Körpers, um den Menschen vor schwerem Schaden durch die Umgebung oder Überbelastung des Körpers zu bewahren. Im Gegensatz dazu hat der chronische Schmerz, der länger anhält als die Ursache des Schmerzes und der erwartungsgemäße Zeitrahmen der Heilung keine bekannte biologische Funktion und betrifft Hunderte von Millionen Menschen weltweit. Rund 7,5 Mio. Menschen leiden allein in der Bundesrepublik Deutschland an chronischen Schmerzen. Unglücklicherweise ist die pharmakologische Behandlung des chronischen Schmerzes noch unbefriedigend und bleibt daher eine Herausforderung für die aktuelle medizinische Forschung. Die derzeit existierenden Analgetika sind häufig nicht ausreichend wirksam und haben z. T. schwere Nebenwirkungen.

Daher werden vielfach neue Targets, körpereigene Strukturen, über die eine schmerzmodulierende Einwirkung, beispielsweise niedermolekularer Wirkstoffe oder anderer Verbindungen wie Antisense Oligodesoxynukleotide (ODN), möglich erscheint, für die Behandlung insbesondere chronischer Schmerzen gesucht. Der von Caterina et al. (1997) klonierte Vanilloid Rezeptor Subtyp 1 (VR1, auch als Capsaicin Rezeptor bezeichnet) ist ein vielversprechender Ansatzpunkt für die Entwicklung neuer Schmerzmedikamente. Es handelt sich dabei um einen Kationenkanal, der vorwiegend durch primäre sensorische Neuronen exprimiert wird (Catarina et al. 1997). VR1 wird durch Capsaicin, eine Komponente der Chilischoten, Hitze (>43°C) und einen niedrigen pH-Wert (Protonen) infolge von Gewebeverletzungen aktiviert und bewirkt einen Calcium-Einstrom in primäre Afferenzen. VR1-Knockout Mäuse entwickelten keine thermische Hyperalgesie nach Gewebeschäden bzw. Entzündungen (Caterina et al., 2000; Davis et al., 2000).

Antisense Oligodesoxynukleotide (ODN), Ribozyme und andere katalytische Nukleinsäuren können zur Behandlung, insbesondere des chronischen Schmerzes durch Abbau oder Veränderung der mRNA ausgewählter Targets - im Falle dieser Erfindung der vorangehend beschriebenen Vanilloid-Rezeptors Subtypes 1 (VR1) (Catarina et al. 1997) - eingesetzt werden, deren Expresssion herunterregulieren und damit die Anzahl der Rezeptoren pro Zelle verringern. Die ODN lagern sich an die mRNA an und blockieren so zum einen die Translation und initiieren zum anderen den Abbau der mRNA durch RNase H, die RNA in einer DNA/RNA Duplex spaltet. Porreca et al. (1999) konnten zeigen, dass intrathekal applizierte ODNs gegen den PN3/SNS-Kanal bei Ratten die Entwicklung von Hyperalgesie und Allodynie durch chronische Nerven- oder Gewebeschädigung verhindern.

Auch wenn die Sequenz des VR1 bekannt ist, so kommt es doch für eine effektive Blockierung und Spaltung der mRNA insbesondere auf die Auswahl der richtigen Antisense-Oligodesoxynukleotide, Ribozyme und anderen katalytische Nukleinsäuren an. Die mRNA des Targets ist meist gefaltet und nur an wenigen Stellen für eine Anlagerung und nachfolgende Spaltung zugänglich. Über die richtige Auswahl der ODN ist aber im Stand der Technik nichts bekannt.

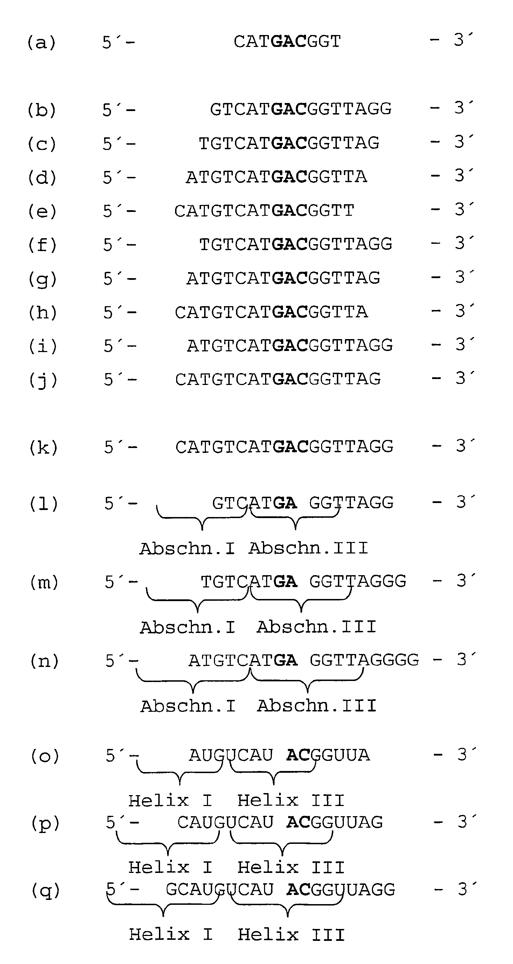

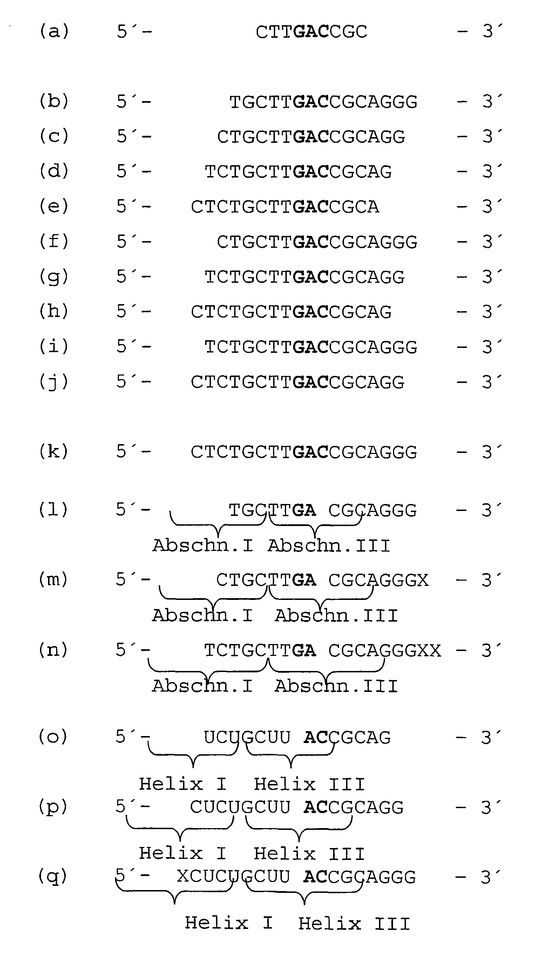

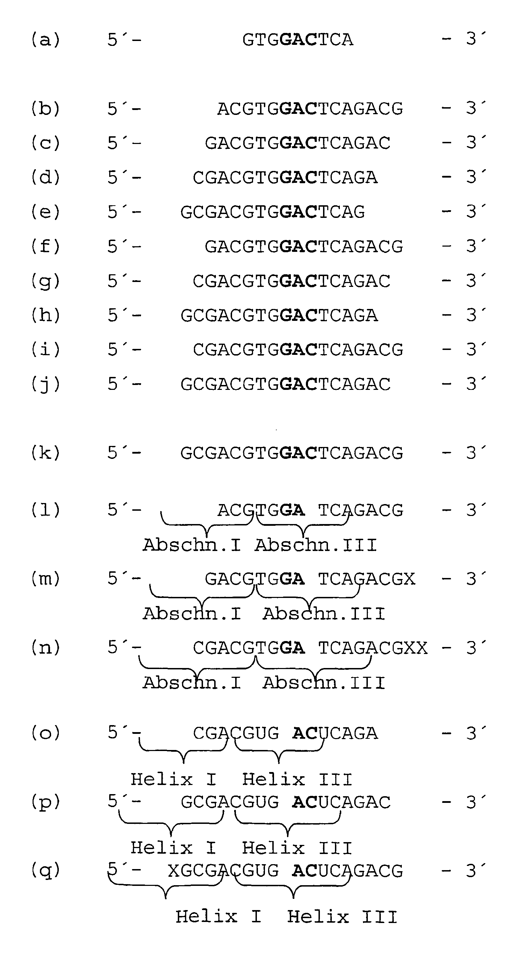

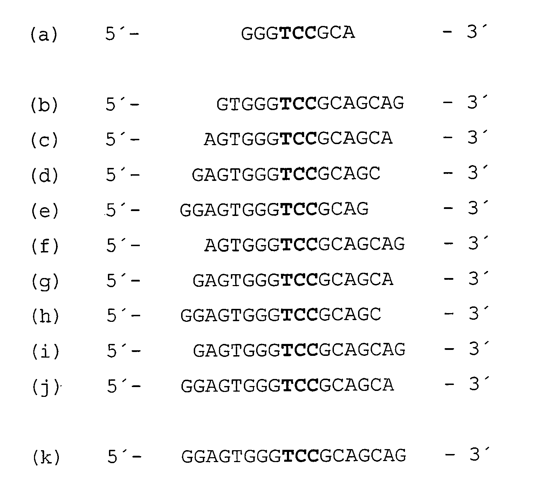

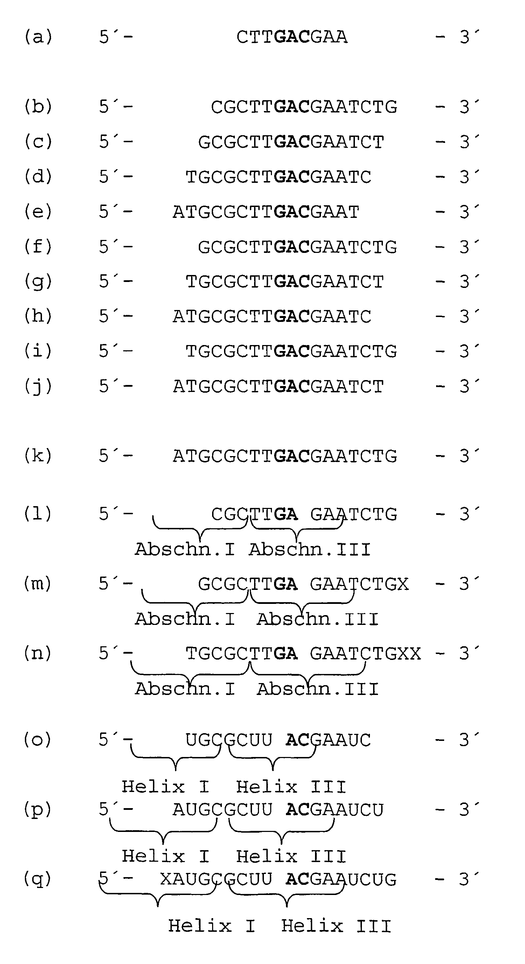

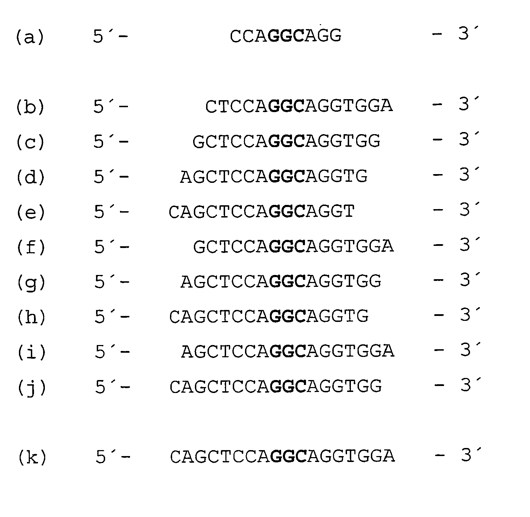

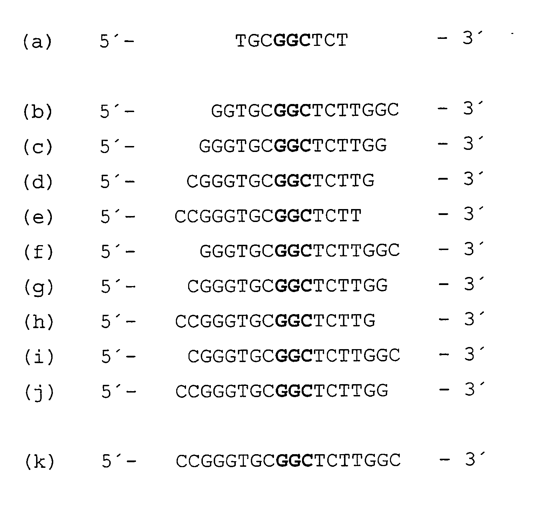

Aufgabe der vorliegenden Schrift war entsprechend die Entwicklung von Antisense Oligodesoxynukleotiden und katalytischer Nukleinsäuren sowie entsprechender Ribozyme gegen die mRNA des Vanilloid Rezeptors. Ein Gegenstand der Erfindung ist daher ein Oligonukleotid enthaltend oder entsprechend eine(r) Basensequenz gemäß einem der Unterpunkte (b) bis (j) in einer der Abbildungen 1/27, 2/27, 4/27, 5/27, 6/27, 7/27, 8/27, 9/27, 10/27, 11/27, 12/27, 13/27, 14/27, 15/27 oder 16/27 oder eine(r) sich in maximal einer abweichenden Base davon unterscheidende(n) Sequenz, wobei sich die Abweichung in der Base nicht im im Unterpunkt (a) abgebildeten Sequenzbereich befindet. Dabei bedeutet Oligonukleotid im Sinne dieser Erfindung ein Molekül mit zwischen 15 und 30 Nukleotiden.

Ein weiterer Gegenstand der Erfindung ist ein Oligonukleotid enthaltend oder entsprechend eine(r) Basensequenz gemäß einem der Unterpunkte (b) bis (j) in einer der Abbildungen 1/27, 2/27, 3/27, 4/27, 5/27, 6/27, 7/27, 8/27, 9/27, 10/27, 11/27, 12/27, 13/27, 14/27, 15/27 oder 16/27.

Ein weiterer Gegenstand der Erfindung ist ein Oligonukleotid enthaltend oder entsprechend eine(r) Basensequenz gemäß Unterpunkt (k) in einer der Abbildungen 1/27, 2/27, 4/27, 5/27, 6/27, 7/27, 8/27, 9/27, 10/27, 11/27, 12/27, 13/27, 14/27, 15/27 oder 16/27 oder eine(r) sich in maximal zwei abweichenden Basen, vorzugsweise einer, davon unterscheidende(n) Sequenz, wobei sich die Abweichurig(en) in der(n) Base(n) nicht im im Unterpunkt (a) abgebildeten Sequenzbereich befindet(n).

Ein weiterer Gegenstand der Erfindung ist ein Oligonukleotid enthaltend oder entsprechend eine(r) Basensequenz gemäß Unterpunkt (k) in einer der Abbildungen 1/27, 2/27, 3/27, 4/27, 5/27, 6/27, 7/27, 8/27, 9/27, 10/27, 11/27, 12/27, 13/27, 14/27, 15/27 oder 16/27.

Ein bevorzugter Gegenstand der Erfindung ist auch ein erfindungsgemäßes Oligonukleotid gemäß einer der vorgenannten erfindungsgemäßen Formen der Oligonukleotide, bei dem das Oligonukleotid ein Länge von 15 bis 30, vorzugsweise 15 bis 25, insbesondere 17 bis 19 oder genau 18, Nukleotiden aufweist.

Ein bevorzugter Gegenstand der Erfindung ist ein erfindungsgemäßes Oligonukleotid das mindestens eine modifizierte oder unmodifizierte Ribose, mindestens eine modifizierte oder unmodifizierte Phosphodiesterbindung und/oder mindestens eine modifizierte oder unmodifizierte Base aufweist.

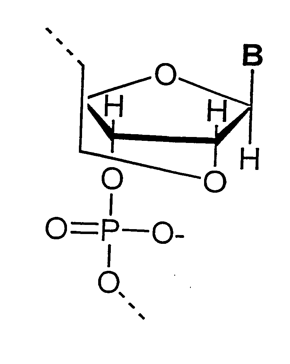

Ein ganz besonders bevorzugter Gegenstand der Erfindung ist ein erfindungsgemäßes Oligonukleotid, bei dem mindestens eines der Nukleotide, insbesondere mehrere der Nukleotide, "Locked Nucleic Acids" ("LNA's") sind oder mindestens eines der Nukleotide, insbesondere alle Nukleotide, Phosphorothioate sind, vorzugsweise ein solches, bei dem mehrere der Nukleotide "Locked Nucleic Acids" ("LNA's") sind. "Locked nucleic acids" ("LNA's") sind Ribonukleotide, die eine Methylen-Brücke enthalten, die den 2'-Sauerstoff der Ribose mit dem 4'-Kohlenstoff verbindet (s. Abb. 27). Einen Überblick über die LNA's geben Braasch D.A. und Corey, D.R. (2001), Locked nucleic acids (LNA); fine-tuning the recognition of DNA und RNA. Chem. Biol. 8, 1-7. Dieser Artikel ist ausdrücklich Mitbestandteil der vorliegenden Beschreibung und Offenbarung. LNA's werden beispielsweise von der Firma Proligo, Boulder, CO, USA angeboten. Auch Phosphorothiate sind dem Fachmann bekannt und können beispielsweise bei MWG-Biotech AG, Ebersberg, Germany bestellt werden.

Bevorzugt sind hierbei Oligonukleotide, in denen die "LNA's" am 5'- und 3'-Ende des Oligonukleotids liegen, vorzugsweise jeweils die letzten 2-5 Nukleotide, insbesondere jeweils die letzten 3 oder 4 Nukleotide, am 3'-und 5'-Ende des Oligonukleotids "LNA's" sind,
und/oder
in denen > 6, insbesondere ≥ 8 zusammenhängende Nukleotide im Oligonukleotid keine "LNA's" sind, vorzugsweise bei denen von den im Sequenzbereich gemäß jeweiligen Unterpunkt (a) abgebildeten Nukleotiden des Oligonukleotids gemäß einer der Abbildungen 1 bis 16 Unterpunkt (b) bis (k) nur jeweils höchstens eines oder keines der Nukleotide eine "LNA" ist.

Bei den mit LNA's oder Phosphorothioaten modifizierten Oligonukleotiden ist es besonders bevorzugt, wenn es sich um ein erfindungsgemäßes Oligonukleotid A oder erfindungsgemäßes Oligonukleotid B, vorzugsweise ein erfindungsgemäßes Oligonukleotid B (s.o.).

Generell ist ein besonderer separater Gegenstand der Erfindung Nukleinsäuren, insbesondere Oligopeptide, bei denen mehrere der Nukleotide "Locked Nucleic Acids" ("LNA's") sind, in denen die "LNA's" am 5'- und 3'-Ende des Oligonukleotids liegen, vorzugsweise jeweils die letzten 2-5 Nukleotide, insbesondere jeweils die letzten 3 oder 4 Nukleotide, am 3'-und 5'-Ende des Oligonukleotids "LNA's" sind, und/oder in denen > 6, insbesondere ≥ 8 zusammenhängende Nukleotide im Oligonukleotid keine "LNA's" sind. Die bisher bezüglich der LNA's ausgeführten Ausführungsformen gelten auch für diesen Gegenstand.

Ein weiterer bevorzugter Gegenstand ist auch ein Polynukleotid-Konstrukt enthaltend mindestens ein erfindungsgemäßes Oligonukleotid. Hier ist Polynukleotidkonstrukt in einem sehr weiten Sinne zu verstehen. Es umfaßt RNA und DNA und Nukleotide ab einer Länge von mindestens 20 Nukleotiden. Dabei versteht man unter (rekombinantes) Polynukleotid-Konstrukt eine generelle Bezeichnung für jede Art von DNA- bzw. RNA-Molekülen, die durch die in vitro-Verknüpfung von DNA-, RNA-Molekülen entstanden sind. Unter Polynukleotid versteht man folgendes: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynukleotid aus mehreren Nukleotiden, die über Phosphorsäure-Pentose-Veresterung miteinander verknüpft sind. Unter die Erfindung fallen aber auch modifizierte Polynukleotide, die zwar die Basenabfolge beibehalten, aber über ein modifiziertes Rückrat statt der Phosphorsäure-Pentose verfügen.

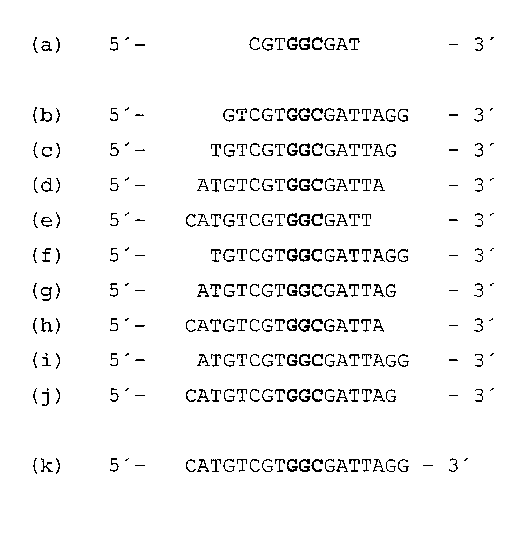

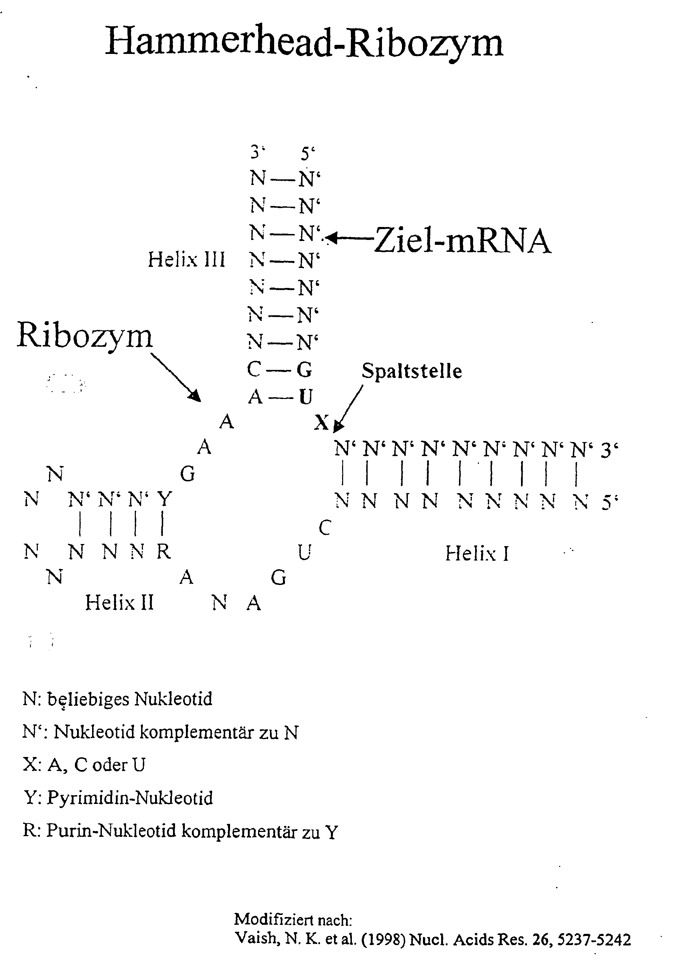

Ein weiterer bevorzugter Gegenstand ist auch ein Polynukleotid-Konstrukt enthaltend in zwei voneinander getrennten Bereichen die beiden Nukleotidteilsequenzen Abschn. I und Abschn. III gemäß einem der Unterpunkte (I) - (n) oder die beiden Nukleotidteilsequenzen Helix I und Helix III gemäß einem der Unterpunkte (o) - (q) in einer der Abbildungen 1/27, 3/27, 4/27, 5/27, 6/27, 7/27, 8/27, 9/27, 11/27, 12/27, 13/27, oder 15/27 oder von diesen Nukleotidteilsequenzen jeweils maximal in einer Base abweichende Nukleotidteilsequenzen. Genaueres über die Aufteilung der beiden Bereiche kann man der Fig. 24) (Helix I und Helix III /Ribozym) und bei Santoro et al. (1997) FIG.2 S. 4264 (Abschnitt I und Abschnitt III/DNA-Enzym) entnehmen.

Ein weiterer bevorzugter Gegenstand ist auch ein Polynukleotid-Konstrukt kodierend für mindestens ein erfindungsgemäßes Oligonukleotid. Darunter ist insbesondere eine abzulesende DNA oder ein Vektor enthaltend DNA oder RNA ist, dessen Produkt ein erfindungsgemäßes Oligonukleotid ist oder sein kann.

Ein weiterer bevorzugter Gegenstand ist auch ein erfindungsgemäßes Polynukleotid-Konstrukt, bei dem es sich um ein Ribozym, ein DNA-Enzym, einen Vektor, insbesondere einen Expressionsvektor, oder eine PNA handelt.

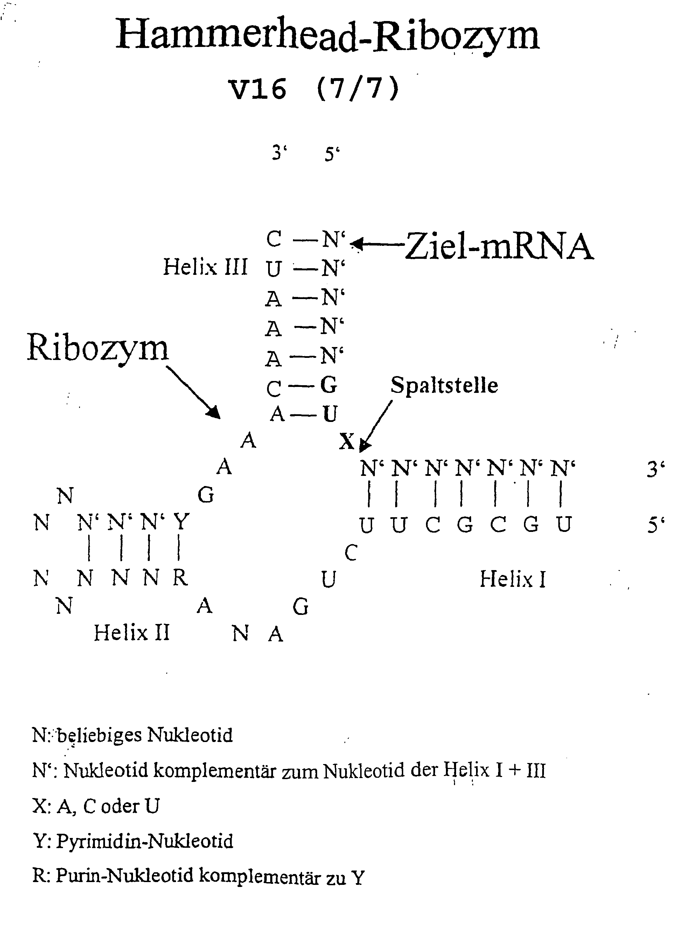

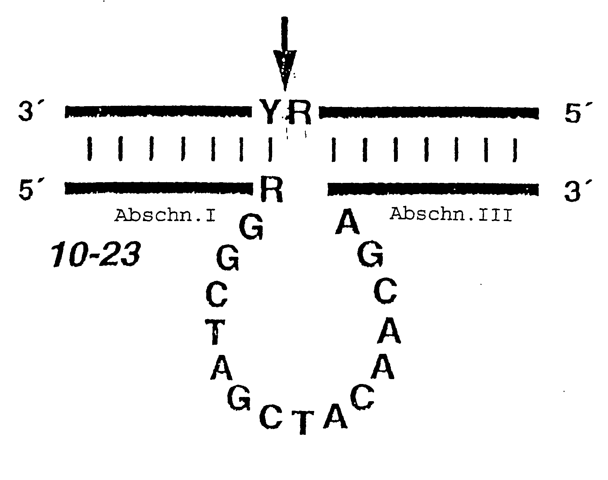

Dabei heißt generell im Sinne dieser Erfindung::
- (Klonierungs)vektor: Generelle Bezeichnung für Nukleinsäure-Moleküle, die beim Klonieren als Träger von Fremdgenen oder Teilen dieser Gene dienen.
- Expressionsvektor: Bezeichnung für speziell konstruierte Klonierungsvektoren, die nach Einbringen in eine geeignete Wirtszelle die Transcription und Translation des in den Vektor einklonierten Fremdgens erlauben.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz: Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease), s. z.B. Hammerhead-Ribozyme gemäß Abb. 24 oder 25 sowie die Beschreibung der Abbildungen oder s. Vaish, N.K. et al. (1998), Nucl. Acid Res. 26, 5237 - 5242.
- DNA-Enzym: Bezeichnung für ein DNA-Molekül, das katalytische Aktivität beinhaltet (z.B. Ligase, Endonuklease, Polymerase, Exonuklease), s. z.B- DNA-Enzym 10-23 gemäß Abb. 26 sowie die Beschreibung der Abbilsdung oder s. Santaro und Joyce (1997) Proc. Natl. Acad. Sci. USA 94, 4262-4266.
- katalytische RNA/DNA: generelle Bezeichnung für Ribozyme bzw. DNA-Enzyme (s.o.).

Ein weiterer bevorzugter Gegenstand ist auch ein erfindungsgemäßes Polynukleotid-Konstrukt enthaltend in zwei voneinander getrennten Bereichen die beiden Nukleotidteilsequenzen-wie oben beschrieben, wobei es sich um ein Ribozym, vorzugsweise ein "hammerhead" Ribozym, oder ein DNA-Enzym, vorzugsweise ein DNA-Enzym vom Typ 10-23 oder 12-32, handelt. Hier ist es besonders bevorzugt und ausgewählt, wenn es sich um ein DNA-Enzym enthaltend mindestens die Nukleotidteilsequenzen Abschn. I und Abschn. III gemäß einem der Unterpunkte (I) bis (n), vorzugsweise (n), in einer der Abbildungen 1/27, 3/27, 4/27, 5/27, 6/27, 7/27, 8/27, 9/27, 11/27, 12/27 oder 13/27,
- vorzugsweise 1/27, 3/27, 7/27 oder 11/27, insbesondere 1/27 oder 3/27, oder
- vorzugsweise 4/27, 6/27, 8/27 oder 12/27, insbesondere 4/27 oder 12/27 handelt. Ebenso ist es besonders bevorzugt und ausgewählt, wenn es sich bei dem Polynukleotid-Konstrukt um ein Ribozym enthaltend mindestens die Nukleotidteilsequenzen Helix I und Helix III gemäß einem der Unterpunkte (o) bis (q), vorzugsweise (o), in einer der Abbildungen 1/27, 3/27, 4/27, 5/27, 6/27, 8/27, 11/27 oder 12/27,
- vorzugsweise 1/27, 3/27 oder 11/27, insbesondere 11/27, oder
- vorzugsweise 4/27, 6/27, 8/27 oder 12/27 insbesondere 4/27 oder 12/27 handelt.

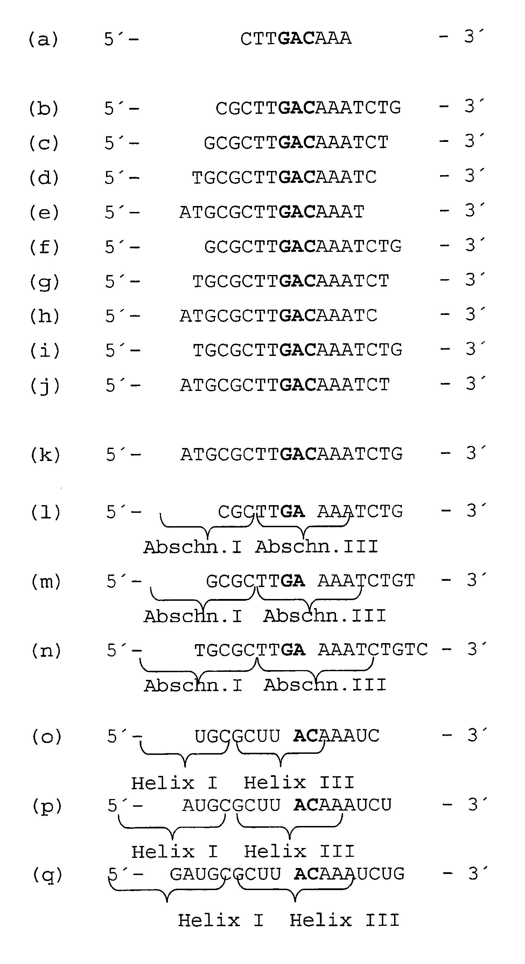

Konkret ist eine bevorzugte Ausführungsform insbesondere Fig 24/27 und 25/27 zu entnehmen. Fig. 24/27 zeigt in allgemeiner Darstellung ein "Hammerhead"-Ribozym nach Vaish, N.K. et al. (1998), Nucl. Acid Res. 26, 5237 - 5242 mit den "erkennenden Armen" Helix I und Helix III, in die jeweils die erfindungsgemäß Helices I und III gemäß der Unterpunkte (o) -(q) in einer der Abbildungen 1/27, 3/27, 4/27, 5/27, 6/27, 7/27, 8/27, 9/27, 11/27, 12/27, 13/27 oder 15/27 eingefügt werden, um zu den erfindungsgemäßen "Hammerhead"-Ribozymen zu kommen. Dabei ersetzt der Abschnitt Helix I in jeweils einer der Abbildungen 1/27, 3/27, 4/27, 5/27, 6/27, 7/27, 8/27, 9/27, 11/27, 12/27, 13/27 oder 15/27 die beliebigen Nukleotide in Helix I nach Abbildung 24/27, so daß das erste Nukleotid am 3'-Ende von Helix I in jeweils einer der Abbildungen 1/27, 3/27, 4/27, 5/27, 6/27, 7/27, 8/27, 9/27, 11/27, 12/27, 13/27 oder 15/27 das erste beliebige Nukleotid "N" am 3'-Ende der Helix I der Abbildung 24/27 ersetzt und die folgenden beliebigen Nukleotide "N" in Helix I Abb. 24/27 in Richtung 5'-Ende durch die Nukleotide ersetzt werden, die in einem, der Unterpunkte (o) bis (q) Helix I in jeweils einer der Abbildungen 1/27, 3/27, 4/27, 5/27, 6/27, 7/27, 8/27, 9/27, 11/27, 12/27, 13/27, oder 15/27 gezeigt sind. Die Nukleotide A" und "C" am 5'-Ende der Helix III in einer der Abbildungen 1/27, 3/27, 4/27, 5/27, 6/27, 7/27, 8/27, 9/27, 11/27, 12/27, 13/27 oder 15/27 ersetzen jeweils die Nukleotide "A" und "C" in Helix III in Abb. 24/27 und die folgenden beliebigen Nukleotide "N" in Helix III Abb. 24/27 werden in Richtung 5'-Ende durch die Nukleotide ersetzt, die in einem der Unterpunkte (o)-(q) Helix III in jeweils einer der Abbildungen 1/27, 3/27, 4/27, 5/27, 6/27, 7/27, 8/27, 9/27, 11/27, 12/27, 13/27 oder 15/27 gezeigt sind. Ein konkretes Beispiel zeigt Abbildung 25/27. Das "Hammerhead"-Ribozyms V16 (7/7) geht auf Abbildung 24/27) und Abbildung 11/27) zurück. Dabei heißt Ribozym V16 (7/7), daß das Enzym gegen die GUC-Site des Oligos V16 gerichtet ist und in den "erkennenden Armen" jeweils 7 Nukleotide (Helix I und Helix III) enthält, hier gemäß Helix I und Helix III von Unterpunkt (o) in Fig. 11). Entsprechendes gilt für alle Ribozyme gemäß Unterpunkten (o bis q) in jeweils einer der Abbildungen 1/27, 3/27, 4/27, 5/27, 6/27, 7/27, 8/27, 9/27, 11/27, 12/27, 13/27, oder 15/27.

Konkret ist eine bevorzugte Ausführungsform insbesondere Fig 26/27 zu entnehmen. Fig. 26/27 zeigt in allgemeiner Darstellung ein DNA-Enzym des Typs '10-23' gemäß Santoro et al., 1997, Fig. 2, S. 4264. Der obere, mit einem Pfel gekennzeichnete Strang ist der zu spaltende RNA-Strang, wobei der Pfeil die Spaltstelle angibt und der untere ist eine Darstellung des DNA-Enzyms. In Bezug auf die vorliegende Anmeldung ist dabei im oberen Strang das "Y" = "U" und das "R" = "G", wobei 3'-wärts vom "Y" ein "C" liegt. Die Spaltstelle auf dem oberen Strang ist daher eine sogenannte GUC-Site (s.o.). Entsprechend ist "R" im unteren Strang = "A" und 5'-wärts vom "R" im unteren Strang befindet sich entsprechend ein "G". Dem schließen sich 5-wärts die weiteren Nukleotide aus Abschnitt I gemäß den Unterpunkten (l bis n) in jeweils einer der Abbildungen 1/27, 3/27, 4/27, 5/27, 6/27, 7/27, 8/27, 9/27, 11/27, 12/27, 13/27 oder 15/27 an, d.h. 5 weitere Nukleotide bei Unterpunkt I, 6 weitere Nukleotide bei Unterpunkt m und 7 weitere Nukleotide bei Unterpunkt n. In Abschnitt III gemäß Abbildung 25/27 - dem zweiten mit der RNA basengepaarten Abschnitt - schließen sich dann direkt an das am Abschnitt III anliegende ungepaarte "A" aus 5'-Richtung 3'-wärts die Nukleotide aus Abschnitt III gemäß den Unterpunkten (l bis n) in jeweils einer der Abbildungen 1/27, 3/27, 4/27, 5/27, 6/27, 7/27, 8/27, 9/27, 11/27, 12/27, 13/27 oder 15/27 an, d.h. 7 weitere Nukleotide bei Unterpunkt I. 8 weitere Nukleotide bei Unterpunkt m und 9 weitere Nukleotide bei Unterpunkt n. Abschnitt III und Abschnitt I sind die sogenannten "erkennenden Arme" des DNA-Enzyms (s. später folgendes Beispiel 3). Das im Rahmen der Erfindung besonders bevorzugte DNA-Enzym des Typs "10-23" für Unterpunkt (n) nach Abb. 1/27 hätte damit folgende Sequenz, wobei der unterstrichene Abschnitt mit der RNA basengepaart wäre:
ATGTCATGA (=R) -GGCTAGCTACAACGA-GGTTAGGGG

Dieses DNA-Enzym würde als V15 (9/9) bezeichnet werden, wobei die Bezeichnung aussagt, daß das Enzym gegen die GUC-Site des Oligos V15 gerichtet ist und in den "erkennenden Armen" jeweils 9 Nukleotide (Abschnitt I und III) enthält, z. B. gemäß Abschnitt I und Abschnitt III von Unterpunkt (n) in Fig. 1). Entsprechendes gilt für alle DNA-Enzyme gemäß Unterpunkten (I bis n) in jeweils einer der Abbildungen 1/27, 3/27, 4/27, 5/27, 6/27, 7/27, 8/27, 9/27, 11/27, 12/27, 13/27 oder 15/27.

Ein weiterer bevorzugter Gegenstand ist auch ein erfindungsgemäßes Polynukleotid-Konstrukt, wobei dieses mindestens eine modifizierte oder unmodifizierte Ribose, mindestens eine modifizierte oder unmodifizierte Phosphodiesterbindung und/oder mindestens eine modifizierte oder unmodifizierte Base aufweist.

Ein weiterer bevorzugter Gegenstand ist auch ein erfindungsgemäßes Oligonukleotid oder erfindungsgemäßes Polynukleotid-Konstrukt, wobei dieses an einen Träger, insbesondere Protein, vorzugsweise tet-, Transportin oder Ferritin; gebunden und/oder in einem Liposom verpackt ist.

Ein weiterer bevorzugter Gegenstand ist auch eine Zelle enthaltend mindestens ein erfindungsgemäßes Oligonukleotid und/oder ein erfindungsgemäßes Polynukleotid-Konstrukt.

Ein weiterer bevorzugter Gegenstand ist auch ein Arzneimittel enthaltend mindestens ein erfindungsgemäßes Oligonukleotid, ein erfindungsgemäßes Polynukleotid-Konstrukt und/oder eine erfindungsgemäße Zelle sowie gegebenenfalls geeignete Hilfs- und/oder Zusatzstoffe. Die erfindungsgemäßen Arzneimittel können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden und enthalten neben den mindestens einem erfindungsgemäßen Gegenstand je nach galenischer Form gegebenenfalls Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Gegenstände in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Gegenstände verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblichweise werden 2 bis 500 mg/kg wenigstens eines erfindungsgemäßen Gegenstandes appliziert. Wenn das Arzneimittel insbesondere zur Gentherapie verwendet werden soll, empfehlen sich als geeignete Hilfs- oder Zusatzstoffe beispielsweise eine physiologische Kochsalzlösung, Stabilisatoren, Proteinase-, DNAse-Inhibitoren etc.

Ein weiterer bevorzugter Gegenstand ist auch ein Diagnostikum enthaltend mindestens ein erfindungsgemäßes Oligonukleotid, ein erfindungsgemäßes Polynukleotid-Konstrukt und/oder eine erfindungsgemäße Zelle sowie gegebenenfalls geeignete Zusatzstoffe.

Dabei bedeuten
- Arzneimittel: ein Stoff entsprechend der Definition im Artikel 1 §2 des deutschen Gesetzes über den Verkehr mit Arzneimitteln (AMG). Das heißt, Stoffe oder Zubereitungen aus Stoffen, die dazu bestimmt sind, durch Anwendung am oder im menschlichen oder tierischen Körper
   1. Krankheiten, Leiden, Körperschäden oder krankhafte Beschwerden zu heilen, zu lindern, zu verhüten oder zu erkennen,
   2. die Beschaffenheit, den Zustand oder die Funktionen des Körpers oder seelische Zustände erkennen zu lassen,
   3. vom menschlichen Körper erzeugte Wirkstoffe oder Körperflüssigkeiten zu ersetzen,
   4. Krankheitserreger, Parasiten oder körperfremde Stoffe abzuwehren, zu beseitigen oder unschädlich zu machen oder
   5. die Beschaffenheit, den Zustand oder die Funktionen des Körpers oder seelische Zustände zu beeinflussen.
- Diagnostikum: Verbindung oder Verfahren, das verwendet werden kann, um eine Krankheit zu diagnostizieren

Ein weiterer bevorzugter Gegenstand ist auch die Verwendung mindestens eines erfindungsgemäßen Oligonukleotid, eines erfindungsgemäßen Polynukleotid-Konstrukt und/oder einer erfindungsgemäßen Zelle zur Herstellung eines Arzneimittels zur Behandlung von Schmerz, insbesondere von chronischem Schmerz, taktiler Allodynie, thermisch ausgelöste Schmerz, und/oder Entzündungsschmerz.

Ein weiterer bevorzugter Gegenstand ist auch die Verwendung mindestens eines erfindungsgemäßen Oligonukleotids, eines erfindungsgemäßen Polynukleotid-Konstrukts und/oder einer erfindungsgemäßen Zelle zur Herstellung eines Arzneimittels zur Behandlung von Harninkontinenz; auch von neurogenen Blasensymptornen; Pruritus, Tumoren, Entzündungen; insbesondere von VR1-Rezeptor-assoziierten Entzündungen mit Symptomen wie Asthma; sowie von allen mit VR1 zusammenhängenden Krankheitssymptomen.

Ein weiterer bevorzugter Gegenstand ist auch ein Verfahren zur Identifizierung schmerzmodulierender Substanzen, dadurch gekennzeichnet, daß die Identifizierung über eine Quantifizierung der Bindung mindestens eines, vorzugsweise markierten, erfindungsgemäßen Oligonukleotids oder mindestens eines erfindungsgemäßen Polynukleotid-Konstrukts an eine RNA erfolgt.

Ein weiterer bevorzugter Gegenstand ist auch ein Verfahren zur Identifizierung schmerzmodulierender Substanzen mit folgenden Verfahrensschritten:
(a) gentechnische Manipulation mindestens einer Zelle (Testzelle) mit mindestens einem erfindungsgemäßen Oligonukleotid und/oder einem erfindungsgemäßen Polynukleotid-Konstrukt,
(a) parallele gentechnische Manipulation mindestens einer identischen Zelle (Kontrollzelle) entweder
   - unterbleibend,
   - unter Durchführung der Manipulation parallel ohne Oligonukleotid oder Polynukleotid-Konstrukt oder
   - mit einem veränderten, nicht mehr erfindungsgemäßen Oligonukleotids oder Polynukleotidkonstrukts,
(b) parallele Inkubation einer zu testenden Substanz unter geeigneten Bedingungen mit mindestens einer Test-Zelle und mindestens einer Konrollzelle und/oder einer Präparation aus solchen Zelle, die mindestens ein Rezeptorprotein, ausgewählt aus der Vanilloid-Rezeptor-Familie, vorzugsweise den VR-1-Rezeptor, synthetisiert hat
(c) Messung der Bindung der Testsubstanz an dem von den Zellen synthetisierten Protein oder Messung mindestens eines der durch die Bindung der Testsubstanz an das Rezeptorprotein veränderten funktionellen Parameter,
(d) Identifizierung der Substanzen über das Ausmaß der Differenz zwischen dem Meßwert bei der Testzelle und dem bei der Kontrollzelle.

Dabei bezieht sich der Begriff schmerzmodulierend auf einen potentiellen regulierenden Einfluß auf das physiologische Schmerzgeschehen, insbesondere auf eine analgetische Wirkung. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper. Die Inkubation unter geeigneten Bedingungen ist hier so zu verstehen, daß die zu untersuchende Substanz mit der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Rezeptor. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Rezeptor aufgrund seiner Erfahrung, der Literatur oder weniger, einfacher Vorversuche leicht festgelegt werden, um im Verfahren einen möglichst deutlichen Meßwert zu erhalten. Eine Zelle, die einen Reuzeptor synthetisiert hat, ist ein Zelle, die diesen Rezeptor bereits endogen exprimiert hat oder eine solche, die gentechnisch verändert wurden, so daß sie diesen Rezeptor exprimiert und entsprechend vor Beginn des erfindungsgemäßen Verfahrens den Rezepror enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Homogenate aus den Zellen, das Cytosol, eine Membranfraktion der Zellen mit Membranfragmenten, eine Suspension isolierter Zellorganellen etc.

Der Maßstab über den das Verfahren die Auffindung interessanter Substanzen erlaubt, ist entweder die Bindung an den Rezeptor, die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Rezeptor. Diese Wechselwirkung kann insbesondere in einer Regulation, Hemmung und/oder Aktivierung von Rezeptoren, Ionenkanälen und/oder Enzymen liegen und veränderte funktionelle Parameter können beispielsweise die Genexpression, das Ionenmilieus, der pH oder das Membranpotentials, bzw. die Veränderung der Enzymaktivität oder der Konzentration der 2^{nd} messenger sein. Dabei heißt:
- gentechnisch manipuliert: Manipulation von Zellen, Geweben oder Organismen derart, daß hier genetisches Material eingebracht wird
- endogen exprimiert: Expression eines Proteins, die eine Zelllinie unter geeigneten Kulturbedingungen aufweist, ohne das dieses entsprechende Protein durch gentechnische Manipulation zur Expression veranlasst wurde

Eine weitere bevorzugter Ausführungsform dieses Verfahrens sieht vor, daß die Zelle bereits vor den Verfahrensschritten (a) und (a') gentechnisch manipuliert wird.

Eine weitere bevorzugter Ausführungsform dieses Verfahrens sieht vor, daß die gentechnische Manipulation die Messung mindestens eines der durch die Testsubstanz veränderten funktionellen Parameter erlaubt.

Eine weitere bevorzugter Ausführungsform dieses Verfahrens sieht vor, daß durch die gentechnische Manipulion eine in der Zelle nicht endogen exprimierte Form eines Mitglieds der Vanilloid-Rezeptor-Familie, vorzugsweise der VR-1-Rezeptor, exprimiert oder ein Reportergen eingeführt wird.

Eine weitere bevorzugter Ausführungsform dieses Verfahrens sieht vor, daß die Messung der Bindung über die Verdrängung eines bekannten markierten Liganden eines Mitglieds der Vanilloid-Rezeptor-Familie, vorzugsweise des VR-1-Rezeptors, erfolgt.

Eine weitere bevorzugter Ausführungsform dieses Verfahrens sieht vor, daß zwischen den parallelen Verfahrensschritten (a) und (a') und dem Verfahrensschritt (b) ≥ 8 h, vorzugsweise ≥ 12 h, insbesondere ≥ 24 h, vergehen.

Ein weiterer bevorzugter Gegenstand ist auch ein Verfahren zur Diagnose von Krankheitsbildern, die mit veränderter Expression von Genen der Vanilloid-Rezeptor-Familie verbunden sind, dadurch gekennzeichnet, daß die Diagnose über eine Quantifizierung der Bindung eines erfindungsgemäßen Oligonukleotids und/oder mindestens eines erfindungsgemäßen Polynukleotid-Konstrukts an eine RNA erfolgt.

Die Oligonukleotide und auch Polynukleotidkonstrukte werden nach dem Fachmann bekannten Verfahren hergestellt. Dabei werden Nukleotide, insbesondere auch Oligonukleotide, beispielsweise nach Art der Merryfield-Synthese, an einem unlöslichen Träger (H.G. Gassen et al., Chemical and Enzymatic Synthesis of Genefragments (Verlag Chemie, Weinheim 1982)) oder auf andere Art synthetisiert (Beyer/Walter; Lehrbuch der Organischen Chemie, 20. Auflage, (S. Hirzel Verlag, Stuttgart 1984), S. 816 ff.).

Ein weiterer Gegenstand der Erfindung ist ein Verfahren zur Behandlung, insbesondere Schmerzbehandlung, eines nichthumanen Säugetieres oder Menschen, das oder der eine Behandlung von Schmerzen, insbesondere chronischer Schmerzen, benötigt, durch Verabreichung eines erfindungsgemäßen Arzneimittels, insbesondere solche enthaltend eine erfindungsgemäßes Oligonukleotid und/oder ein erfindungsgemäßes Polynukleotid-Konstrukt. Ein weiterer Gegenstand sind auch entsprechende Verfahren zur Behandlung von Pruritus und/oder Harninkontinenz.

Die folgenden Beispiele und Abbildungen sollen die Erfindung erläutern, ohne daß der Gegenstand der Erfindung darauf beschränkt wäre.

### Abbildungen und Beispiele

### Abbildungen

Abbildungen, Abb.. Figur und Fig. sind als synonym zu betrachten. Ebenso synonym sind im Zusammenhang mit den Abbildungen Subtyp und Unterpunkt. Ein "X" in den abgebildeten Sequenzen steht für ein beliebiges zur entsprechenden Base auf der mRNA von VR1 komplementäres Nukleotid. Die Abbildungen zeigen:

### Figur 1/27

Generell Nukleotidsequenzen ausgehend von einer Antisense Oligodesoxynukleotidsequenz gegen die mRNA des VR1 aus der Ratte, hier häufig als Oligo V15, Oligonukleotid Nr. 15 oder V15 bezeichnet. Unterpunkte (a) -(j) zeigen verkürzte Ausschnitte aus dieser Antisense-Oligodesoxynukleotidsequenz, Unterpunkt (k) zeigt die volle Antisense-Oligodesoxynukleotidsequenz, mit der das "messenger walk screening" durchgeführt wurde, Unterpunkte (l) - (n) zeigen jeweils zwei (Abschn. I und Abschn. III) von der vollen Antisense-Oligodesoxynukleotidsequenz ausgehende Nukleotidteilsequenzen, die jeweils nicht überlappende Teilbereiche dieser Seqenz umfassen oder diesen entsprechen, meist an oder in der GAC-Region getrennt sind und in bestimmten Polynukleotidkonstrukten, insbesondere DNA-Enzymen, in zwei voneinander getrennten Bereichen, den erkennenden Armen" auftreten, Unterpunkte (o) - (q) zeigen jeweils zwei (Helix I und Helix III) von der vollen Antisense-Oligodesoxynukleotidsequenz ausgehende Nukleotidteilsequenzen (hier allerdings als RNA), die jeweils nicht überlappende Teilbereiche dieser Seqenz umfassen oder diesen entsprechen, meist an oder in der GAC-Region getrennt sind und in bestimmten Polynukleotidkonstrukten, insbesondere Ribozymen, in zwei voneinander getrennten Bereichen, den "erkennenden Armen" auftreten.

### Figur 2/27

Die dem Oligo V15 in Figur 1/27 in der Position auf der mRNA entsprechende Sequenz eines Antisense-Desoxyoligonukleotids gegen humanes VR1. Die Untertypen (a) - (k) entsprechen bezüglich Art und generellem Inhalt den bereits zu Figur 1/27 beschriebenen.

### Figur 3/27

Generell Nukleotidsequenzen ausgehend von einer Antisense Oligodesoxynukleotidsequenz gegen die mRNA des VR1 aus der Ratte, hier häufig als Oligo V30, Oligonukleotid Nr. 30 oder V30 bezeichnet. Art und Inhalt der Untertypen entspricht den bereits zu Figur 1 beschriebenen.

### Figur 4/27

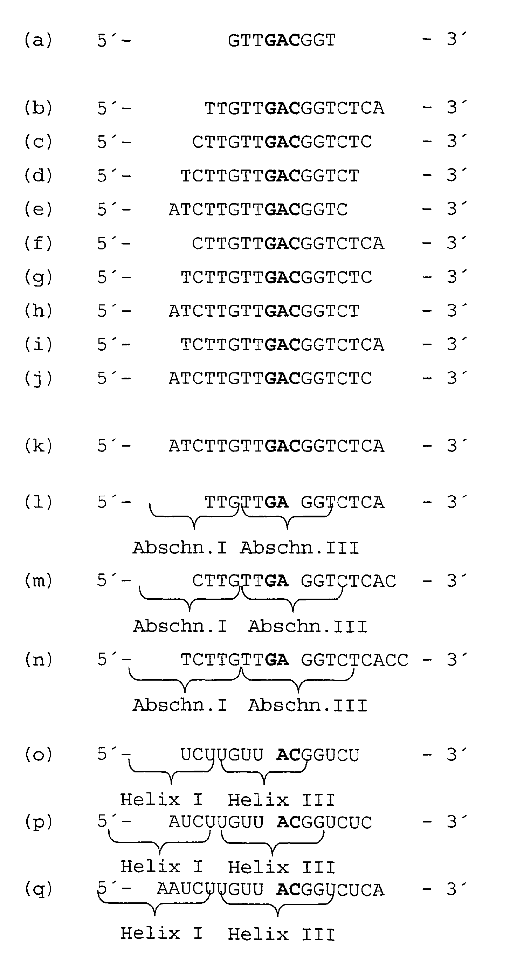

Die dem Oligo V30 in Figur 3/27 in der Position auf der mRNA entsprechende Sequenz eines Antisense-Desoxyoligonukleotids gegen humanes VR1. Die Untertypen (a) - (q) entsprechen bezüglich Art und generellem Inhalt den bereits zu Figur 1/27 beschriebenen.

### Figur 5/27

Generell Nukleotidsequenzen ausgehend von einer Antisense Oligodesoxynukleotidsequenz gegen die mRNA des VR1 aus der Ratte, hier häufig als Oligo V32, Oligonukleotid Nr. 32 oder V32 bezeichnet. Art und Inhalt der Untertypen entspricht den bereits zu Figur 1/27 beschriebenen.

### Figur 6/27

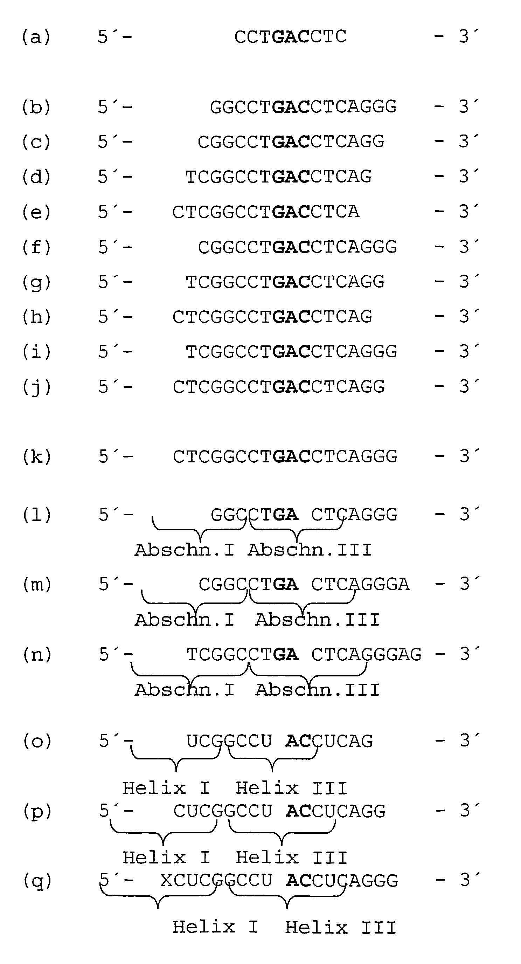

Die dem Oligo V32 in Figur 5/27 in der Position auf der mRNA entsprechende Sequenz eines Antisense-Desoxyoligonukleotids gegen humanes VR1: Die Untertypen (a) - (q) entsprechen bezüglich Art und generellem Inhalt den bereits zu Figur 1/27 beschriebenen.

### Figur 7/27

Generell Nukleotidsequenzen ausgehend von einer Antisense Oligodesoxynukleotidsequenz gegen die mRNA des VR1 aus der Ratte, hier häufig als Oligo V26, Oligonukleotid Nr. 26 oder V26 bezeichnet. Art und Inhalt der Untertypen entspricht den bereits zu Figur 1/27 beschriebenen.

### Figur 8/27

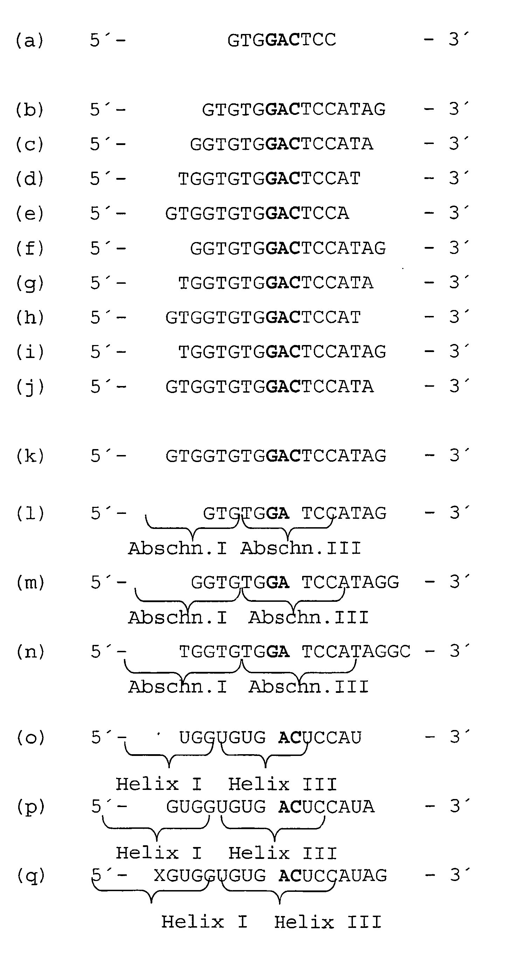

Die dem Oligo V26 in Figur 7/27 in der Position auf der mRNA entsprechende Sequenz eines Antisense-Desoxyoligonukleotids gegen humanes VR1. Die Untertypen (a) - (q) entsprechen bezüglich Art und generellem Inhalt den bereits zu Figur 1/27 beschriebenen.

### Figur 9/27

Generell Nukleotidsequenzen ausgehend von einer Antisense Oligodesoxynukleotidsequenz gegen die mRNA des VR1 aus der Ratte, hier häufig als Oligo V2, Oligonukleotid Nr. 2 oder V2 bezeichnet. Art und Inhalt der Untertypen entspricht den bereits zu Figur 1/27 beschriebenen.

### Figur 10/27

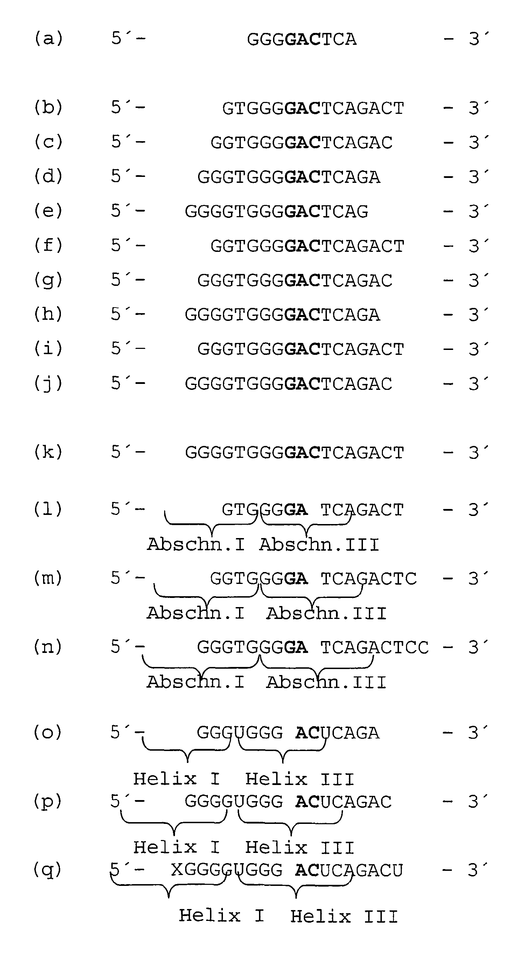

Die dem Oligo V2 In Figur 9/27 in der Position auf der mRNA entsprechende Sequenz eines Antisense-Desoxyoligonuklevtids gegen humanes VR1. Die Untertypen (a) - (k) entsprechen bezüglich Art und generellem Inhalt den bereits zu Figur 1/27 beschriebenen.

### Figur 11/27

Generell Nukleotidsequenzen ausgehend von einer Antisense Oligodesoxynukleotidsequenz gegen die mRNA des VR1 aus der Ratte, hier häufig als Oligo V16, Oligonukleotid Nr. 16 oder V16 bezeichnet. Art und Inhalt der Untertypen entspricht den bereits zu Figur 1/27 beschriebenen.

### Figur 12/27

Die dem Oligo V16 in Figur 11/27 in der Position auf der mRNA entsprechende Sequenz eines Antisense-Desoxyoligonukleotids gegen humanes VR1. Die Untertypen (a) - (q) entsprechen bezüglich Art und generellem Inhalt den bereits zu Figur 1/27 beschriebenen.

### Figur 13/27

Generell Nukleotidsequenzen ausgehend von einer Antisense Oligodesoxynukleotidsequenz gegen die mRNA des VR1 aus der Ratte, hier häufig als Oligo V28, Oligonukleotid Nr. 28 oder V28 bezeichnet. Art und Inhalt der Untertypen entspricht den bereits zu Figur 1/27 beschriebenen.

### Figur 14/27

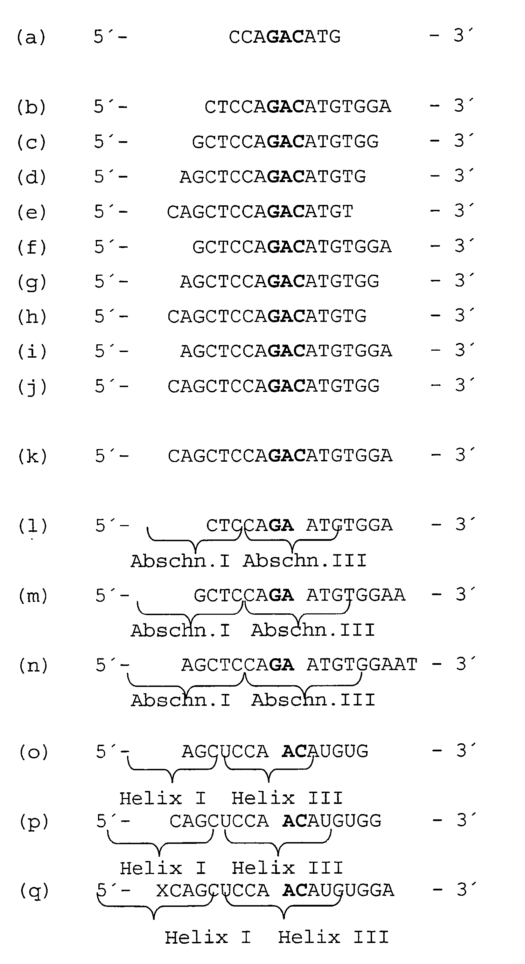

Die dem Oligo V28 in Figur 13/27 in der Position auf der mRNA entsprechende Sequenz eines Antisense-Desoxyoligonukleotids gegen humanes VR1. Die Untertypen (a) - (k) entsprechen bezüglich Art und generellem Inhalt den bereits zu Figur 1/27 beschriebenen.

### Figur 15/27

Generell Nukleotidsequenzen ausgehend von einer Antisense Oligodesoxynukleotidsequenz gegen die mRNA des VR1 aus der Ratte, hier häufig als Oligo V4, Oligonukleotid Nr. 4 oder V4 bezeichnet. Art und Inhalt der Untertypen entspricht den bereits zu Figur 1/27 beschriebenen.

### Figur 16/27

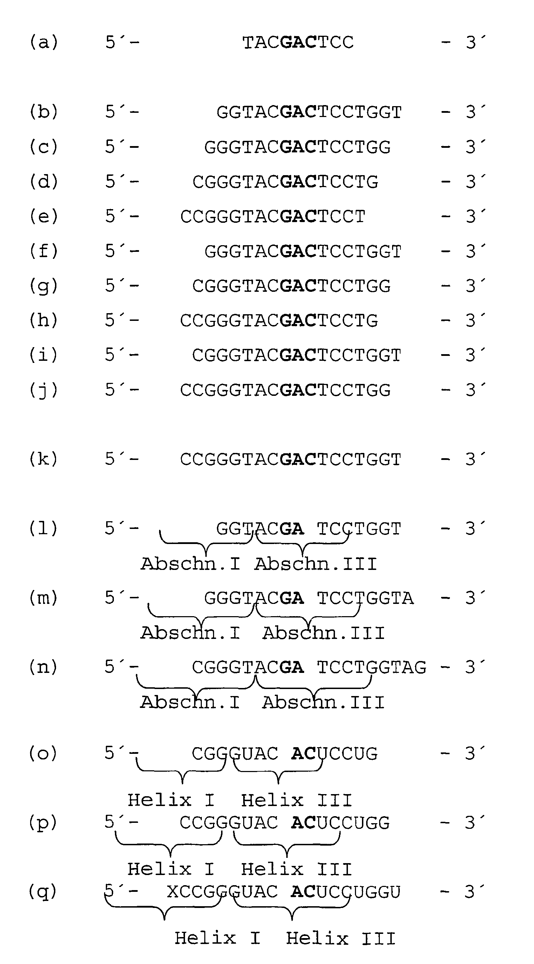

Die dem Oligo V4 in Figur 15/27 in der Position auf der mRNA entsprechende Sequenz eines Antisense-Desoxyoligonukleotids gegen humanes VR1. Die Untertypen (a) - (k) entsprechen bezüglich Art und generellem Inhalt den bereits zu Figur 1/27 beschriebenen.

### Figur 17/27

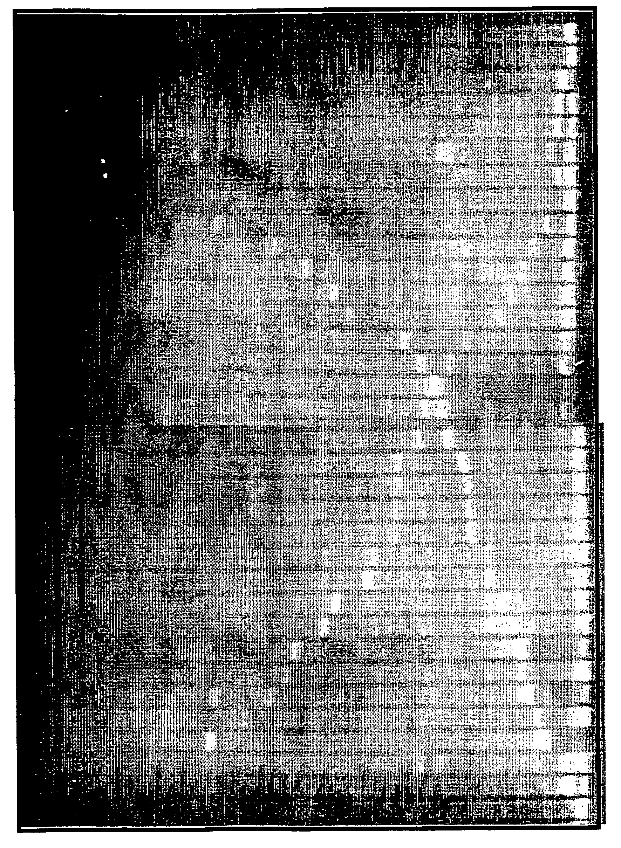

Fig. 17/27 zeigt das Ergebnis des Messenger Walk Screenings. Zu sehen sind in jeder Spur neben der oberen Bande des ungeschnittenen Substrates die zwei Produktbanden der geschnittenen mRNA sowie einige unspezifische Banden. Zu sehen ist die VR1 mRNA nach dem Abbau durch RNase H in der Gegenwart von jeweils einem von 33 Antisense-Oligonukleotiden (Oligo V1 bis Oligo V33). Zu sehen sind in jeder Spur neben der oberen Bande des ungeschnittenen Substrates die zwei Produktbanden der geschnittenen mRNA sowie einige unspezifische Banden.
Spur 1: VR1 mRNA, Spur 2-34: RNase H Assay mit Antisense Oligodesoxynukleotiden gegen die 33 GUC-Sites der VR1 mRNA (Oligo V1 bis Oligo V33).

### Figur 18):

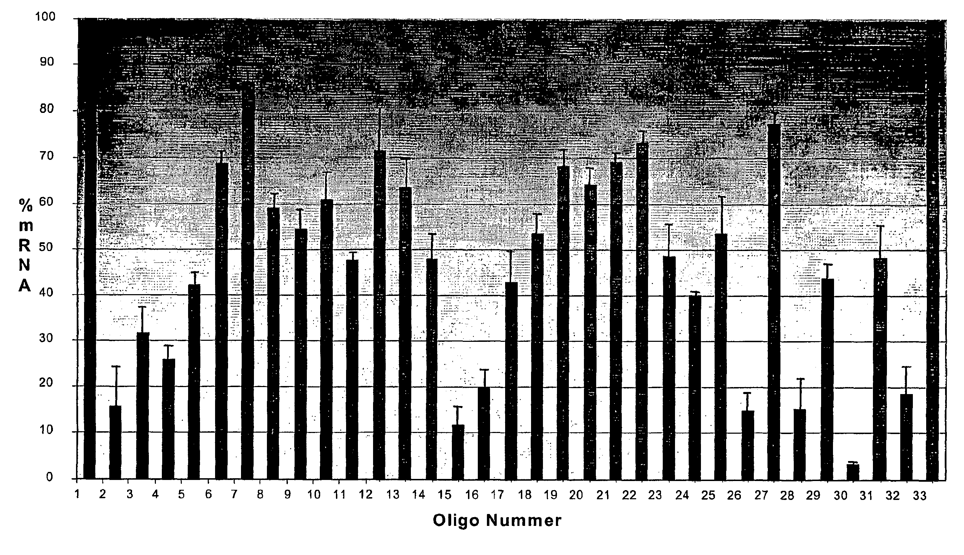

Quantitative Auswertung des Messenger Walk Screenings. In Figur 18/27 ist der prozentuale Anteil der ungeschnittenen mRNA nach dem RNase H Assay mit den einzelnen ODNs aufgetragen. Jeder Wert repräsentiert den Mittelwert aus mindestens drei Experimenten, so daß die Standardabweichung in keinem Fall 10% übersteigt. Die Antisense Oligodesoxynukleotide gegen die 15. und 30. GUC-Site binden am effizientesten an die VR1 mRNA. sodass diese zu 88 ± 4 bzw. 97 ± 1% durch die RNase H abgebaut wird (Oligo V15 und Oligo V30).

### Figur 19/27

Bild von einem Gel nach dem RNase H Assay mit den Oligonukleotiden V15. V15Ktr. (Mismatch), V30 und V30 Ktr. (Mismatch).
Gezeigt sind VR1 mRNA (Spur 1) sowie RNase H Assay mit den Oligodesoxynukleotiden V15, V15Ktr., V30 und V30Ktr (Spur 2-5).

### Figur 20/27

Quantitative Beurteilung des Schneidens der mRNA durch Ribozyme und DNA-Enzyme unter Bedingungen unter "Single turnover"-Bedingungen.

### Figur 21/27

Kinetik des VR1 mRNA-Schneidens durch Ribozyme und DNA-Enzyme unter "Single turnover"-Bedingungen.

### Figur 22/27

Kinetik der VR1 mRNA-Schneidens durch Ribozyme und DNA-Enzyme unter "Multiple turnover"-Bedingungen.

### Figur 23/27

Abschätzung der taktilen Allodynie während der Behandlung durch VR1-Antisense(AS)- und Mismatch(MS)Oligodesoxynukleotide (V15 und V15Ktr.).

### Figur 24/27

Allgemeine Darstellung eines "Hammerhead"-Ribozyms mit den "erkennenden Armen' Helix I und Helix III, in die jeweils die Helices I und III gemäß Unterpunkte (o) - (q) in einer der Abbildungen 1/27, 3/27, 4/27, 5/27, 6/27, 7/27, 8/27, 9/27, 11/27, 12/27, 13/27 oder 15/27 eingefügt werden, um zu den erfindungsgemäßen "Hammerhead"-Ribozymen zu kommen (s. Beschreibung zu Figur 1/27) Dabei ersetzt der Abschnitt Helix in jeweils einer der Abbildungen 1/27, 3/27, 4/27, 5/27, 6/27, 7/27, 8/27, 9/27, 11/27, 12/27, 13/27 oder 15/27 die beliebigen Nukleotide in Helix I nach Abbildung 24/27 so, daß das erste Nukleotid am 3'-Ende von Helix I in jeweils einer der Abbildungen 1/27, 3/27, 4/27, 5/27, 6/27, 7/27, 8/27, 9/27, 11/27, 12/27, 13/27 oder 15/27 das erste beliebige Nukleotid "N" am 3'-Ende der Helix I der Abbildung 24/27 ersetzt und die folgenden beliebigen Nukleotide "N" in Helix I Abb. 24/27 in Richtung 5'-Ende durch die Nukleotide ersetzt werden, die in einem der Unterpunkte (o) bis (q) Helix I in jeweils einer der Abbildungen 1/27, 3/27, 4/27, 5/27, 6/27, 7/27, 8/27, 9/27, 11/27, 12/27, 13/27 oder 15/27 gezeigt sind. Die Nukleotide "A" une "C" am 5'-Ende der Helix III in einer der Abbildungen 1/27, 3/27, 4/27, 5/27, 6/27, 7/27, 8/27, 9/27, 11/27, 12/27, 13/27 oder 15/27 ersetzen jeweils die Nukleotide "A" und "C" in Helix III in Abb. 24/27 und die folgenden beliebigen Nukleotide "N" in Helix III Abb. 24/27 werden in Richtung 5'-Ende durch die Nukleotide ersetzt, die in einem der Unterpunkte (o)-(q) Helix III in jeweils einer der Abbildungen 1/27, 3/27, 4/27, 5/27, 6/27, 7/27, 8/27, 9/27, 11/27, 12/27, 13/27 oder 15/27 gezeigt sind. Ein konkretes Beispiel zeigt Abbildung 25/27.

### Figure 25/27

Darstellung des (besonders bevorzugten) "Hammerhead"-Ribozyms V16 (7/7), nach Abbildung 24/27 und Abbildung 11/27. Dabei heißt Ribozym V16 (7/7), daß das Enzym gegen die GUC-Site des Oligos V16 gerichtet ist und in den "erkennenden Armen" jeweils 7 Nukleotide (Helix I und Helix III) enthält, hier gemäß Helix I und Helix III von Unterpunkt (o) in Fig. 11/27. Entsprechendes gilt für alle Ribozyme gemäß Unterpunkten (o bis q) in jeweils einer der Abbildungen 1/27, 3/27, 4/27, 5/27, 6/27, 7/27, 8/27, 9/27 11/27, 12/27, 13/27 oder 15/27.

### Figur 26/27

Darstellung des DNA-Enzyms des Typs '10-23' gemäß Santoro et al., 1997, Fig. 2, S. 4264:

Der obere, mit einem Pfel gekennzeichnete Strang ist der zu spaltende RNA-Strang, wobei der Pfeil die Spaltstelle angibt und der untere ist eine Darstellung des DNA-Enzyms. In Bezug auf die vorliegende Anmeldung ist dabei im oberen Strang das "Y" = "U" und das "R" = "G", wobei 3'-wärts vom "Y" ein "C" liegt. Die Spaltstelle auf dem oberen Strang ist daher eine sogenannte GUC-Site (s.o.). Entsprechend ist "R" im unteren Strang = "A" und 5'-wärts vom "R" im unteren Strang befindet sich entsprechend ein "G". Dem schließen sich 5-wärts die weiteren Nukleotide aus Abschnitt I gemäß den Unterpunkten (l bis n) in jeweils einer der Abbildungen 1/27, 3/27, 4/27, 5/27, 6/27, 7/27, 8/27, 9/27, 11/27, 12/27, 13/27 oder 15/27 an, d.h. 5 weitere Nukleotide bei Unterpunkt I, 6 weitere Nukleotide bei Unterpunkt m und 7 weitere Nukleotide bei Unterpunkt n. In Abschnitt III gemäß Abbildung 25/27 - dem zweiten mit der RNA basengepaarten Abschnitt - schließen sich dann direkt an das am Abschnitt III anliegende ungepaarte "A" aus 5'-Richtung 3'-wärts die Nukleotide aus Abschnitt III gemäß den Unterpunkten (l bis n) in jeweils einer der Abbildungen 1/27, 3/27, 4/27, 5/27, 6/27, 7/27, 8/27, 9/27, 11/27, 12/27, 13/27 oder 15/27 an, d.h. 7 weitere Nukleotide bei Unterpunkt I, 8 weitere Nukleotide bei Unterpunkt m und 9 weitere Nukleotide bei Unterpunkt n. Abschnitt III und Abschnitt I sind die sogenannten "erkennenden Arme" des DNA-Enzyms (s. Beispiel 3). Das DNA-Enzym des Typs "10-23" für Unterpunkt (n) nach Abb. 1 hätte damit folgende Sequenz, wobei der unterstrichene Abschnitt mit der RNA basengepaart wäre:
ATGTCATGA (=R) - GGCTAGCTACAACGA - GGTTAGGGG

Dieses (besonders bevorzugte) DNA-Enzym würde als V15 (9/9) bezeichnet werden, wobei die Bezeichnung aussagt, daß das Enzym gegen die GUC-Site des Oligos V15 gerichtet ist und in den "erkennenden Armen" jeweils 9 Nukleotide (Abschnitt I und III) enthält, z. B. gemäß Abschnitt I und Abschnitt III von Unterpunkt (n) in Fig. 1). Entsprechendes gilt für alle DNA-Enzyme gemäß Unterpunkten (I bis n) in jeweils einer der Abbildungen 1//27, 3/27, 4/27, 5/27, 6/27, 7/27, 8/27, 9/27, 11/27, 12/27, 13/27 oder 15/27.

### Fig. 27//27

Schematische Darstellung einer "Locked Nucleic Acid" LNA:

### Beispiele:

### Identifizierung der generell geeigneten Schnittstellen:

Der erste Schritt In der Antisense- und Ribozym-Strategie ist die Identifizierung zugänglicher Stellen der mRNA für die Bindung von Oligonukleotiden, insbesondere aber auch von Ribozymen. Dazu mußte die mRNA des VR1 auf diese Schnittstellen hin untersucht werden. Eine Analyse der VR1-mRNA ergab im kodierenden Bereich folgende potentielle Erkennungsstellen für Ribozyme:
33 X GT(U)C-Sequenzen
28 X GT(U)T-Sequenzen
12 X GT(U)A-Sequenzen
Um die zugänglichen Stellen der VR1 mRNA zu ermitteln, wurden in einem ersten Schritt drei unabhängige Nukleotidgemische folgender Sequenz synthetisiert:

| | |
|---|---|
| Gemisch 1: NNNAACNNN | sog. GUU-Library |
| Gemisch 2: NNNCACNNN | sog. GUA-Library |
| Gemisch 3: NNNGACNNN | sog. GUC-Library |

Diese wurden konsekutiv in einem Rnase H-Experiment verwendet und es zeigte sich, daß nur mit der GUC-Library ein nennenswerter Abbau der VR1-mRNA zu beobachten war. Somit sind von den potentiellen Zielsequenzen für Ribozyme die 33 GUC-Sites in der VR1 mRNA am besten zugänglich und wurden zur weiteren Analyse eingesetzt.

### Beispiel 2:

### Identifizierung der effektivsten Antisense Oligodesoxynukleotide Messenger Walk Screening

Zur identifikation von Bereichen der mRNA, die für Antisense Oligodesoxynukleotide zugänglich sind, wurde diese systematisch mit ODNs im RNase H Assay gescreent (Messenger Walk Screening). Die ODNs waren 18 Nukleotide lang und enthielten in der Mitte eine GAC-Sequenz, die revers-komplementär zu GUC-Sequenzen in der mRNA ist. Dieses Triplet wurde als Target gewählt, da es gute Ergebnisse zeigte und in einem zweiten Schritt zur Entwicklung von Hammerhead Ribozymen und DNA Enzymen verwendet werden kann. Insgesamt wurden 33 ODNs, die als V1 bis V33 bezeichnet wurden, gegen sämtliche GUC-Sites der VR1 mRNA getestet. Die ODNs wurden systematisch durch die Zugabe von jeweils einem ODN und RNase H zur mRNA auf ihre Eignung hin gescreent. Die RNase H schneidet gebildete DNA/RNA-Duplexe wo immer ein Oligonukleotide an die mRNA binden kann (Fig. 17/27).

### In vitro Transkription der VR1 mRNA

Zunächst wurde die cDNA des Vanilloid Rezeptors in den Vektor pcDNA3.1 (+) der Firma Invitrogen kloniert. Anschließend erfolgte die *in vitro* Transkription der mRNA mit dem RiboMAX Large Scale RNA Production System - T7 der Firma Promega nach Angaben des Herstellers.

### RNase H Assay

Zum Test, ob ein Antisense Oligodesoxynukleotid an die mRNA gebunden hat, wurde ein RNase H Assay durchgeführt. Dazu wurde die VR1 mRNA (100 mM) mit einem fünffachen Überschuss des ODNs in einem Gesamtvolumen von 10 µl in 40 mM Tris/HCl pH 7,2; 4 mM MgCl₂; 1 mM DTT und 150 mM NaCl für 7,5 min bei 37°C in Gegenwart von 0,4 u RNase H (von Promega) inkubiert. Die Reaktionen wurden durch Zugabe von EDTA (65 mM Endkonzentration) gestoppt. Die Proben wurden über ein 1.5%iges Agarose-Gel aufgetrennt und mit Ethidiumbromid (1 µg/ml) 20 min angefärbt. Die Gele wurden mit dem Gel Doc 2000 Gel Documentation System der Firma Biorad photographiert und mit dem Programm Quantity One ausgewertet.

Fig. 17/27 zeigt das Ergebnis des Messenger Walk Screenings. Zu sehen sind in jeder Spur neben der oberen Bande des ungeschnittenen Substrates die zwei Produktbanden der geschnittenen mRNA sowie einige unspezifische Banden.

Eine Quantifizierung des RNA-Abbaus zeigte, daß durch das effektiveste Antisense Oligonukleotid (Oligo Nr. 30 (V30)) mehr als 90% der VR1 mRNA spezifisch geschnitten/gespalten werden konnte, wenn es im 5-fachem Überschuß gegenüber der Ziel-mRNA eingesetzt wird (Fig. 18/27).

Die Intensitäten der einzelnen Banden wurden ausgewertet. In Figur 18/27 ist für die einzelnen ODNs der Anteil der ungeschnittenen mRNA für den RNase H Assay aufgetragen. Die Antisense Oligodesoxynukleotide gegen die 15. und 30. GUC-Site (Oligo V15 und Oligo V30) binden am effizientesten an die VR1 mRNA, so daß diese zu 88 ± 4 bzw. 97 ± 1% durch die RNase H abgebaut wird.

Die Sequenzen der in diesem Test effektivsten Antisense-Oligodesoxynukleotide finden sich in Abbildungen 1/27, 3/27, 5/27, 7/27, 9/27, 11/27, 13/27 und 15/27, wobei u.a. die als Oligo V15 (Abb. 1) und Oligo V30 (Abb. 3) bezeichneten bevorzugt erscheinen in Betracht gezogen wurden.

Als Mismatch-Kontrollen für diese Antisense Oligodesoxynukleotide wurden für die folgenden Experimente Oligodesoxynukleotide synthetisiert und verwendet, bei denen jede fünfte und sechste Base (bzw. - falls diese identisch sind - zwei benachbarte Basen) vertauscht sind. Die Sequenzen der Kontroll Oligodesoxynukleotide (Mismatch) lauten:
Oligo V15Ktr.: CAT GCT ATG AGC GTT GAG
Oligo V30Ktr.: ATC TGT TTG AGC GTC TAC

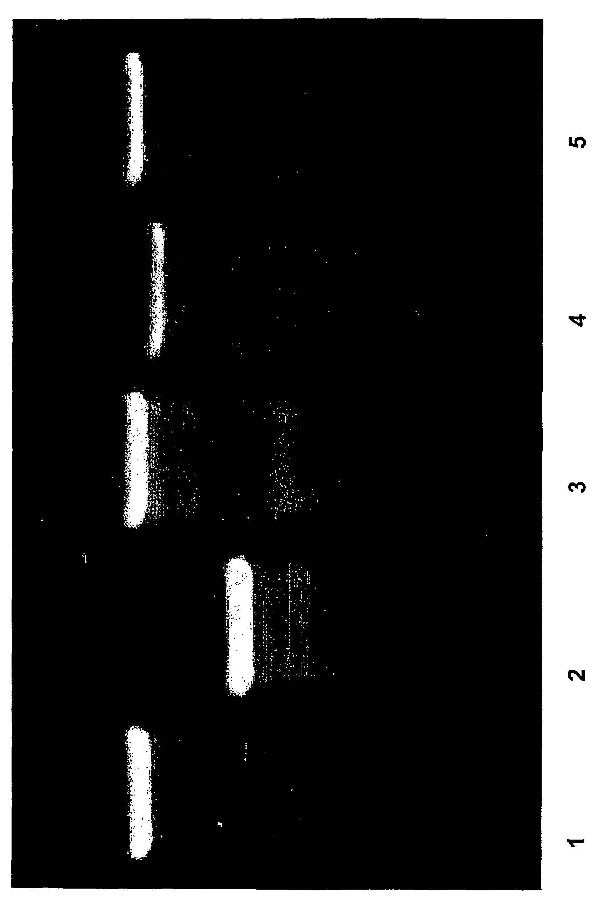

Zur Kontrolle wurde der RNase H Assay mit den Oligonukleotiden V15, V15Ktr., V30 und V30 Ktr. durchgeführt (s. Abb. 19/27). Erwartungsgemäß wird die RNA nur mit den Antisense ODNs, nicht aber mit den Mismatch-Kontrollen abgebaut, da diese nicht an die mRNA binden.

### Zusammenfassung:

Im RNase H Assay mit Antisense Oligodesoxynukleotiden gegen sämtliche GUC-Triplets der VR1 mRNA konnten Oligodesoxynukleotide gegen die GUC-Triplets (2, 4, 15, 16, 26, 28, 30 und 32), insbesondere das 15. und 30. als die am besten bindenden ODNs identifiziert werden. Diese Oligodesoxynukleotide sowie zwei Mismatch-Kontrollen stehen somit für Versuche im Tiermodell und andere Verwendungen zur Verfügung.

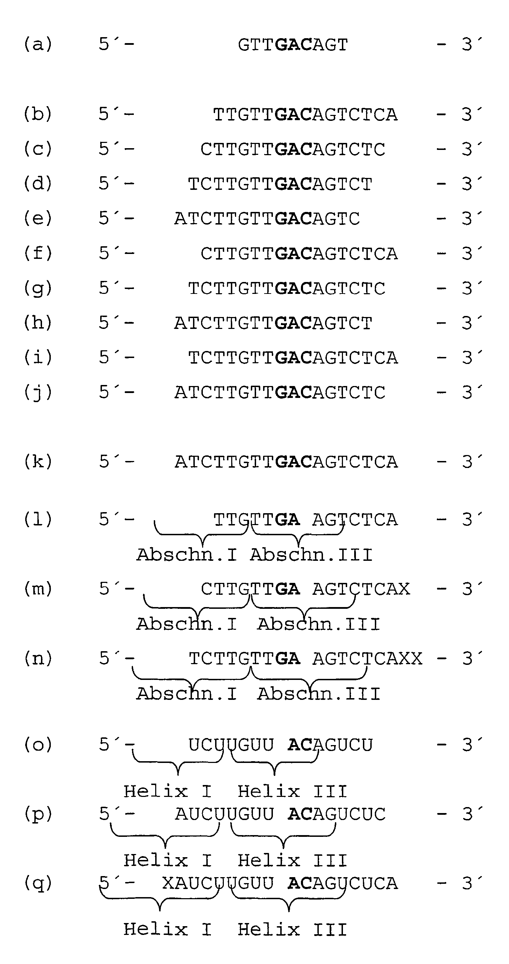

Entsprechendjnteressant sind auch die humanen Sequenzen gem. Abb. 2/27, 4/27, 6/27, 8/27, 10/27, 12/27, 14/27, 16/27, die von der Position den in der Ratte gefundenen entsprechen. Die mRNA der Ratte und des Menschen sind stark homolog (wahrscheinlich auch in der Faltung) und es ist daher klar, daß günstig erreichbare identifizierte Schnittstellen auf der Ratten mRNA auch human interessant sind. Dabei sind die Sequenzen besonders bevorzugt, die auch das GUC-Triplett zeigen, die Abbildungen 4/27, 6/27, 8/27 und 12/27, und aufgrund der der Ratte ähnlichen Lokalisation auch die V15 und V30 entsprechenden gemäß Abbildungen 2/27 und 4/27.

### Beispiel 3:

### Ribozyme und DNA-Enzyme

Weiter wurden aufgrund der gefundenen effektivsten Bindungsstellen entsprechende Ribozyme und DNA-Enzyme untersucht.

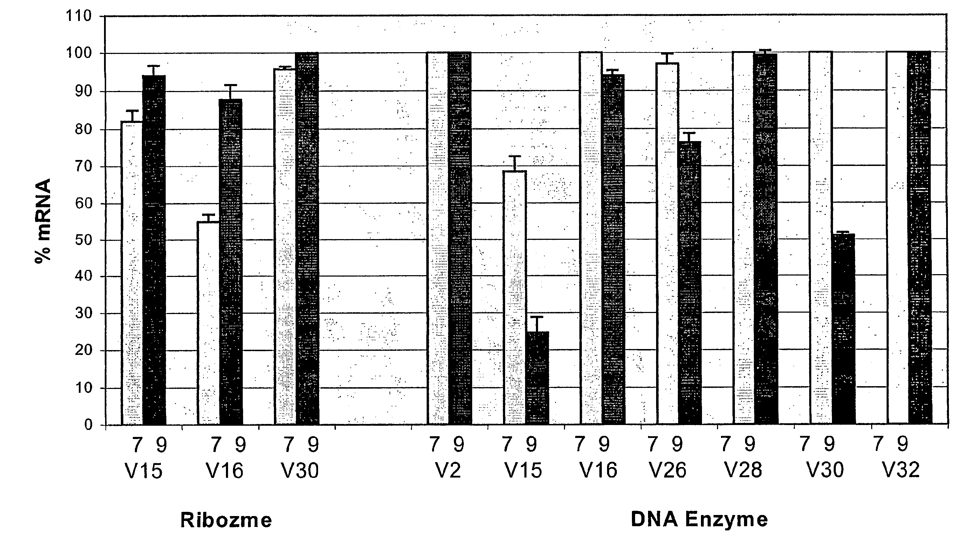

"Hammerhead"-Ribozyme und DNA-Enzyme des Typs '10-23' (Santoro et al., 1997) wurden gegen die Stellen die mRNA konstruiert, welche für ODNs zugänglich waren. Die Länge der "erkennenden Arme" war 7 oder 9 Nukleotide auf jeder Seite. Quantitative Auswertung der mRNA-Spaltung/Schneidung unter "Single turnover"-Bedingungen (10-facher Überschuß an Ribozymen und DNA-Enzymen) zeigte nach 20 min bei 37°C, daß Ribozyme mit kürzeren "erkennenden Armen" (Helix I und Helix III) aktiver sind, während widerum bei DNA-Enzyme solche mit längeren "Armen" (Abschn.I und Abschn. III) aktiver sind (Fig. 20//27). Eine schematische Abbildung eines Ribozyms mit den Helices I und III zeigt Fig. 24/27) und ein Beispiel Fig 25/27). Für ein DNA-Enzym 10-23 kann man dies (5'-wärts (Abschnitt I) und 3'-wärts (Abschnitt III) vom katalytischen Motiv) bei Santoro et al. (1997; S. 4264, Fig.2) entnehmen (s. auch Fig 26) mit der Figurenbeschreibung).

Experimente mit Ribozymen und DNA-Enzymen wurden in 50 mM Tris/HCl pH 7.5 und 10 mM MgCl₂ bei 37°C durchgeführt. Für "Single tumover"-Experimente wurden Ribozyme und DNA-Enzyme im 10-fachen Oberschuß verwendet. Bei "Multiple tumover--Experimente wurde das Substrat mRNA im 10-fachen Überschuß verwendet.

### "Single turnover"-Kinetik

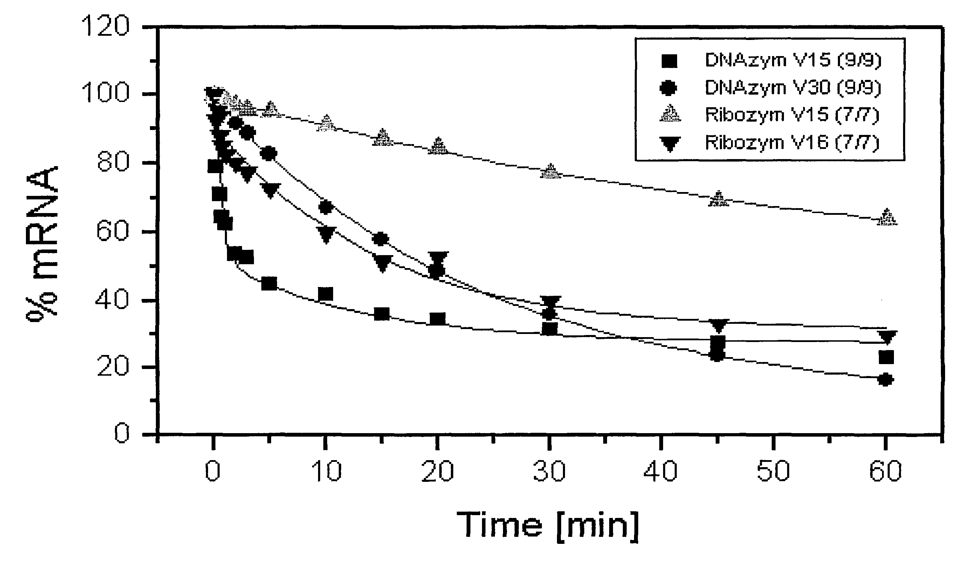

Es wurde eine kinetische Analyse unter "Single turnover"-Bedingungen für die zwei effektivsten Ribozyme und DNA-Enzyme durchgeführt (Fig. 21/27). Die Daten sind in Tabelle 1 zusammengefaßt. DNA-Enzym V15 (9/9) (s. Beschreibung Fig. 26/27), das die mRNA mit einer biphasischen Kinetik schneidet, hat die höchste Rate (Geschwindigkeitskonstante), gefolgt von Ribozym V16 (7/7) (s. Fig 25/27), DNA-Enzym V30 (9/9) und dem langsamsten Ribozym V15 (7/7). Dabei heißt z.B. DNA-Enzym V15 (7/7), daß das Enzym gegen die GUC-Site des Oligos V15 gerichtet ist und in den "erkennenden Armen" jeweils 7 Nukleotide (Abschnitt I und III) enthält, z. B. gemäß Abschnitt I und Abschnitt III von Subtyp (I) in Fig. 1/27).

### Tabelle 1:

**Tab. 1: Kinetische Daten der Ribozyme und DNA-Enzyme gegen VR1 mRNA unter "Single turnover"- Bedingungen**

| | A₁ | k₁ | A₂ | k₂ | A_{∝} |
|---|---|---|---|---|---|
| | | [min⁻¹] | | [min⁻¹] | |
| DNAzym V15 (9/9) | 0,43 ± 0,05 | 2,3 ± 0,5 | 0,34 ± 0,04 | 0,07 ± 0,01 | 0,21 ± 0,03 |
| DNAzym v30 (9/9) | 0,90 ± 0,02 | 0,042 ± 0,002 | - | - | 0,08 ± 0,02 |
| Ribo V15 (7/7) | 0,51 ± 0,09 | 0,023 ± 0,007 | - | - | 0,49 ± 0,09 |
| Ribo V16 (7/7) | 0,58 ± 0,02 | 0,077 ± 0,008 | - | - | 0,34 ± 0,03 |

### "Multiple turnover"-Kinetik

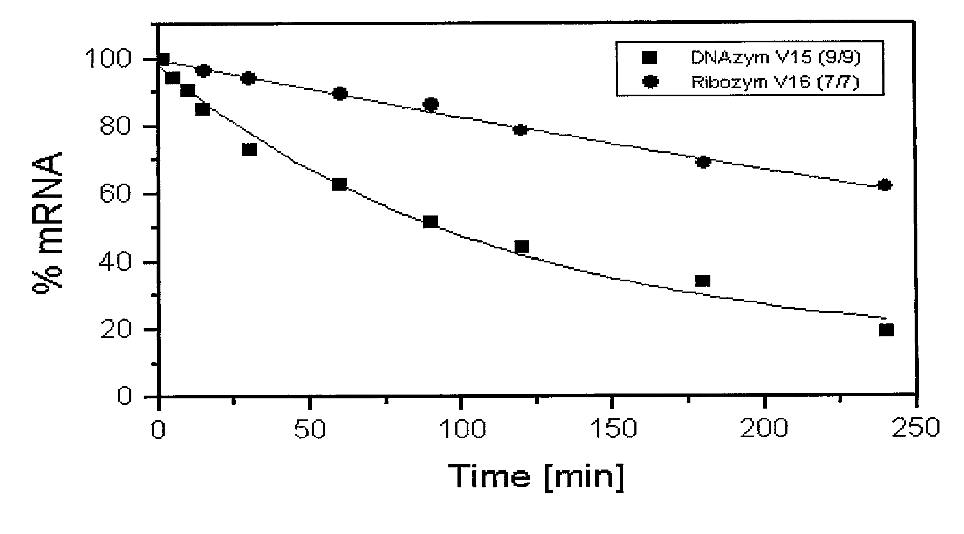

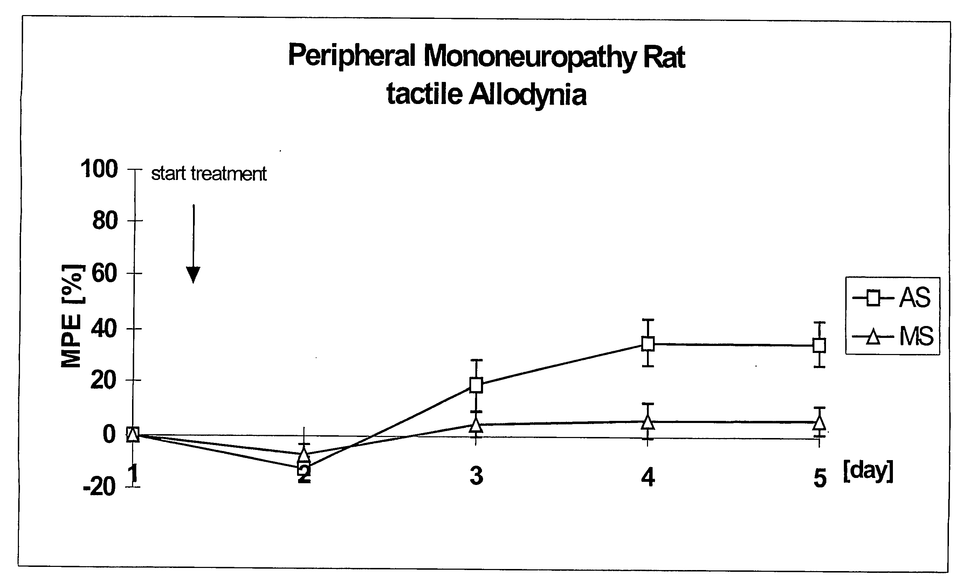

Die Spaltung der mRNA unter "Multiple turnover"-Bedingungen (10-facher Substrat Überschuß) wird in Fig. 22/27 gezeigt. Wieder hat das DNA-Enzym V15 (9/9) eine höhere anscheinende (apparente) Rate ((Geschwindigkeitskonstante) (Faktor 3.4) als das Ribozyme V16 (7/7) (Tab. 2).

### Tabelle 2:

**Tab. 2: Kinetische Daten der Ribozyme und DNA-Enzyme gegen VR1 mRNA unter "Multiple turnover"- Bedingungen.**

| | k |
|---|---|
| | [min⁻¹] |
| DNAzyme V15 (9/9) | (6,5 ± 0,6) * 10⁻³ |
| RiboV16(7/7) | (1,9 ± 0,2) * 10⁻³ |

### Beispiel 4:

### In vivo Experimente

In 20 mänlichen Sprague-Dawley-Ratten wurden an den linken L5/L6 Spinal-Nerven Spinal-Nerven-Ligaturen gemäß Kim und Chung (1992) angelegt. Zur selben Zeit wurden Spinal-Katheter gemäß Pogatzki et al.⁶ (2000) implantiert. 4 bis 6 Tage nach der Operation wurde die taktile Schwellen-Basislinie (Rückzugs-Schwellen/withdrawal threshholds) an der ipsi- und contralateralen Hinterpfote durch ein elektronisches vonFrey-Anesthesiometer (IITC Life Science, USA) gemessen. Der korrekte Sitz der Spinal-Katheter wurde durch Lidocain-Gabe (10µl, 2%) bestätigt, die in einer kurzfristigen Lähmung beider Hinterglieder resultierte. Nach dem Test und Messung der Basislinie wurden je 45µg VR-1 Antisense- (AS, n=10) oder Mismatch (MS, n=10)-Oligonukleotide in 0.9% NaCl einmal am ersten Tag und b.i.d. an den folgende 4 Tagen gegeben. Die taktilen Rückzugs-Schwellen (withdrawal threshholds) wurden 30 Minuten nach der ersten täglichen Oligo-Gabe gemessen. Die Ergebnisse sind als % maximal possible effect (%MPE: % des maximal möglichen Effekts) auf der ipsilateralen Seite angegeben, in dem man die Basisline als 0% und die Rückzugs-Schwelle einer Kontroll-Gruppe als 100%MPE annimmt. Verwendet wurden als Antisense Oligo V15 (Fig. 1/27, Subtyp k) und als Mismatch Oligo V15Ktr. wie oben bereits beschrieben.

Die Behandlung von mononeuropathischen Ratten mit Antisense- aber nicht mit Mismatch-Oligodesoxynukleotiden induzierte eine Verringerung der taktilen Allodynie beginnend am dritten Tag der Behandlung und ein Plateau an den Tagen 4 und 5 der Behandlung erreichend. Es gab keinen Effekt auf die Rückzugs-Schwellen der contralateralen Hinterpfoten.

### Beispiel 5:

### "Locked Nucleotides"/Gegen Abbau geschützte Oligonukleotide bzw. Oligonukleotid-Konstrukte

Es wurden verschiedene erfindungsgemäße Oligonukleotide insbesondere gemäß Untergruppe (k) der Abbildungen 1/27 und z.T. auch Abbildung 3/27 hergestellt. Die meisten von diesen waren LNA-Konstrukte, die bei PROLIGO in Boulder, Co, USA bestellt wurden und bei denen LNA's an verschiedenen Stellen saßen (s. Tabelle 3). Weiter wurde ein unverändertes Oligonukleotid gemäß Untergruppe (k) der Abbildungen 1/27 synthetisiert und ein von der Basensequenz entsprechendes Phosphorothioat bei MWG Biotech AG in Ebersberg Deutschland bestellt.

**Tabelle 3. Liste der verwendeten Oligonukleotide. Kleinbuchstaben= DNA-Monomere, kursiv und unterstrichen = Phosphorothioate, fette Großbuchstaben= LNA monomere.**

| **Name** | **Sequenz** | **% mRNA Spaltung** | **% LNA-Gehalt** | **DNA-Lücke** |
|---|---|---|---|---|
| **DNA 1** | catgtcatgacggttagg | 90 ± 1 | 0 | - |
| **PS** | *CATGTCATGACGGTTAGG* | 71 ± 7 | 0 | - |
| | Gemischte | | | |
| LNA 1 | catg**T**ca**T**gacgg**T**tagg | 12 ± 2 | 16 | 5 |
| LNA 2 | **C**atg**T**ca**T**gacgg**T**tagg | 11 ± 9 | 22 | 5 |
| LNA 3 | catg**T**ca**T**gacgg**TT**agg | 4 ± 1 | 22 | 5 |
| LNA 4 | catg**T**ca**T**gaCgg**T**tag**G** | 5 ± 9 | 27 | 2 |
| LNA 5 | C**a**T**g**T**ca**T**ga**C**gg**TT**ag**G | 2 ± 3 | 44 | 2 |
| LNA 6 | catg**T**cat**G**acgg**T**tagg | 12 ± 2 | 16 | 4 |
| LNA 7 | **C**atg**T**cat**G**acgg**T**tagg | 8 ± 4 | 22 | 4 |
| LNA 8 | catg**T**cat**G**acgg**T**tag**G** | 9 ± 1 | 22 | 4 |
| LNA 9 | **C**atg**T**cat**G**acgg**T**tag**G** | 7 ± 2 | 27 | 4 |
| LNA 10 | **C**atg**T**ca**T**ga**C**gg**T**tag**G** | 7 ± 1 | 33 | 2 |
| LNA 11 | **C**a**T**g**T**catgacgg**TT**ag**G** | 81 ± 5 | 33 | 8 |
| | Lücken | | | |
| LNA 12 | **C**atg**T**ca**T**gacgg**T**tag**G** | 6 ± 4 | 27 | 5 |
| LNA 13 | **C**atg**T**c**A**tgacgg**T**tag**G** | 49 ± 12 | 27 | 6 |
| LNA 14 | **C**atg**T**Catgacgg**T**tag**G** | 83 ± 6 | 27 | 7 |
| LNA 15 | CatgTcatgacggTtagG | 91 ± 2 | 22 | 8 |
| | Endblock Oligonukleotide | | | |
| LNA 16 | **CATGT**catgacgg**TTAGG** | 85 ± 6 | 55 | 8 |
| LNA 17 | **CATG**tcatgacggt**TAGG** | 94 ± 3 | 44 | 10 |
| LNA 18 | **CAT**gtcatgacggtt**AGG** | 93 ± 2 | 33 | 12 |
| LNA 19 | **Ca**tgtcatgacggtta**GG** | 87 ± 12 | 22 | 14 |
| LNA 20 | **C**atgtcatgacggttag**G** | 85 ± 6 | 11 | 16 |
| | controls sequence | | | |
| DNA 2 | atcttgttgacggtctca | 97 ± 2 | 0 | - |
| LNA 21 | **A**tct**T**gtt**G**acgg**T**ctc**A** | 0 ± 0 | 27 | 4 |
| LNA 22 | A**tct**T**gttgacgg**T**ctc**A | 95 ± 3 | 22 | 8 |
| LNA 23 | **ATCTT**gttgacgg**TCTCA** | 97 ± 1 | 55 | 8 |

Mit diesen Oligonukleotiden wurden verschiedene Versuche unternommen:
a) Zunächst wurde der prozentuale Umfang der durch das Oligonukleotid ausgelösten RNA-Spaltung von VR1 durch RNAse H untersucht, wobei die Versuchsbedingungen im wesentlichen mit den in Beispiel 2 genannten Bedingungen übereinstimmten.
Die Ergebnisse sind Tabelle 3 zu entnehmen. Es stellte sich heraus, daß Oligonukleotide mit LNA erst mit dem nativen Oligonukleotid vergleichbare Spaltung zeigten, wenn mindestens 6 oder besser 8 zusammenhängende Nukleotide keine LNA's sind.
b) Dann wurde die Schmelztemperatur der LNA/RNA:DNA Hybride nach Standardmethoden gemessen (Tabelle 4). Überraschenderweise zeigten die LNA's eine erhöhte Schmelztemperatur im Vergleich zu den nativen Oligonukleotiden und den Phosphorothioaten. Das ist sehr günstig für die Stabilität.

**Tabelle 4. Schmelztemperatur Tₘ von LNA/DNA:RNA Hybriden**

| **Oligonukleotide** | **Anzahl der LNA'ₛ** | **Tₘ °C** |
|---|---|---|
| DNA 1 | 0 | 58 |
| PS | 0 | 49 |
| LNA 20 | 2 | 61 |
| LNA 19 | 4 | 66 |
| LNA 18 | 6 | 73 |
| LNA 17 | 8 | 79 |
| LNA 16 | 10 | -85 |

c) Dann wurde die Kinetik der RNASe H-Spaltung unter gleichen Bedingungen wie oben untersucht, nur daß äquimolare Mengen RNA und Antisense Oligonukleotid (je 100 nM) verwendet wurden. Die Ergebnisse sind in Tabelle 5 gezeigt. Überraschenderweise zeigte das Oligonukleotid mit LNA eine deutliche Steigerung der Aktivität im Vergleich zu den nativen Oligonukleotiden und den Phosphorothioaten.

**Tabelle 5. Spaltungsraten-Konstanten für die RNase H-Spaltung von "fulllength" VR1 mRNA induziert durch verschiedene Antisense Oligonukleotide.**

| Oligonukleotide | k |
|---|---|
| | [min⁻¹] |
| DNA 1 | 0.17 ± 0.01 |
| LNA 17 | 1.1 ± 0.6 |
| PS | 0.07 ± 0.01 |

d) Abschließend wurde die Halbwertszeit der Oligonukleotide mit LNA sowie des nativen Oligonukleotids und des Phosphorothioats radioaktiv markiert bei 37°C in humanem Serum über bis zu 2 Tage bestimmt. Die Ergebnisse sind in Tabelle 6 gezeigt. Während die native DNA eine Halbwertszeit von 1,5 h und das Phosphorothioat eine von 10h zeigt, sind die Nukleotide mit LNA, die an den Enden 3 oder 4 LNA's hatten, mit t_{1/2} von ca. 17h deutlich besser.

**Tabelle 6. Halbwertszeit von nativem, Phosphorothioat- und End-block LNA/DNA-Oligonukleotiden in humanem Serum.**

| Oligonukleotide | nt end block | t_{1/2} [h] |
|---|---|---|
| DNA 1 | 0 | 1.5 ± 0.3 |
| PS | 0 | 10 ± 2 |
| LNA 20 | 1 | 4 ± 2 |
| LNA 19 | 2 | 6 ± #?# |
| LNA 18 | 3 | 17 ± 2 |
| LNA 17 | 4 | 15 ± 1 |
| LNA 16 | 5 | 15 ± 2 |

An besten schnitten daher die Nukleotide mit LNA's ab, die ca. 8 zusammenhängende Nukleotide ohne LNA's hatten und am 3'- und 5'-Ende 3 oder 4 LNA's hatten.

### Literatur

Caterina, M. J.; Schumacher, M. A.; Tominaga, T. A.; Rosen, T. A.; Levine, J. D.; Julius, D. (1997) The capsaicin receptor: a heatactivated ion channel in the pain pathway. Nature 389, 816-824.
Caterina, M. J.; Leffler, A.; Malmberg, A. B.; Martin, W. J.; Trafton, J.; Petersen-Zeitz, K. R.; Koltzenburg, M; Basbaum, A. I.; Julius, D. (2000) Impaired nociception and pain sensation in mice lacking the capsaicin receptor. Science 288, 306-313.
Davis, J. B.; Gray, J.; Gunthorpe, M. J.; Hatcher, J. P.; Davey, P. T.; Overend, P.; Harries, M. H.; Latcham, J.; Clapham, C.; Atkinson, K.; Hughes, S. A.; Rance, K.; Grau, E.; Harper, A. J.; Pugh, P. L.; Rogers, D. C.; Bingham, S.; Randall, A.; Sheardown, S. A. (2000) Vanilloid receptor-1 is essential for inflammatory thermal hyperalgesia. Nature 405, 183-187.
Kim, S. H.; Chung, J. M. (1992) An experimental model for peripheral mononeuropathy produced by segmental spinal nerve ligation in the rat. Pain 50, 355-363.
Pogatzki, E. M.; Zahn, P. K.; Brennan, T. J. (2000) Lumbar catheterization of the subarachnoid space with a 32-gauge polyurethane catheter in the rat. Eur. J. Pain 4, 111-113.
Porreca, F.; Lai, J.; Bian, D.; Wegert, S.; Ossipov, M. H.; Eglen, R. M.; Kassotakis, L.; Novakovic, S.; Rabert, D. K.; Sangameswaran, L.; Hunter, J. C. (1999) A comparison of the potential role of the tetrodotoxin-insensitive sodium chanels, PN3/SNS and NaN/SNS2, in rat models of chronic pain. Proc. Natl. Acad. Sci. USA 96, 7640-7644.
Santoro, S. W.; Joyce, G. F. (1997) A general purpose RNA-cleaving DNA enzyme. Proc. Natl. Acad. Sci. USA 94, 4262-4266.
Vaish, N.K. et al. (1998), Nucl. Acid Res. 26, 5237 - 5242.

## Patentansprüche

1. Oligonukleotid enthaltend oder entsprechend eine(r) Basensequenz gemäß einem der Unterpunkte (b) bis (j) in einer der Abbildungen 1/27, 2/27, 3/27, 4/27, 5/27, 6/27, 7/27, 8/27, 9/27, 10/27, 11/27, 12/27, 13/27, 14/27, 15/27 oder 16/27 oder eine(r) sich in maximal einer abweichenden Base davon unterscheidende(n) Sequenz, wobei sich die Abweichung in der Base nicht im im Unterpunkt (a) abgebildeten Sequenzbereich befindet, **dadurch gekennzeichnet, daß** das Oligonukleotid eine Länge von 15 bis 30, vorzugsweise 15 bis 25, insbesondere 17 bis 19 oder genau 18, Nukleotiden aufweist.

2. Oligonukleotid enthaltend oder entsprechend eine(r) Basensequenz gemäß Unterpunkt (k) in einer der Abbildungen 1/27, 2/27, 3/27, 4/27, 5/27, 6/27, 7/27, 8/27, 9/27, 10/27, 11/27, 12/27, 13/27, 14/27, 15/27 oder 16/27 oder eine(r) sich in vorzugsweise einer und maximal zwei abweichenden Basen davon unterscheidende(n) Sequenz, wobei sich die Abweichung(en) in der(n) Base(n) nicht im im Unterpunkt (a) abgebildeten Sequenzbereich befindet(n), **dadurch gekennzeichnet, daß** das Oligonukleotid eine Länge von 15 bis 30, vorzugsweise 15 bis 25, insbesondere 17 bis 19 oder genau 18, Nukleotiden aufweist.

3. Oligonukleotid gemäß einem der Ansprüche 1 oder 2, **dadurch gekennzeichnet, daß** es mindestens eine modifizierte oder unmodifizierte Ribose, mindestens eine modifizierte oder unmodifizierte Phosphodiesterbindung und/oder mindestens eine modifizierte oder unmodifizierte Base aufweist.

4. Oligonukleotid gemäß einem der Ansprüche 1 bis 3, **dadurch gekennzeichnet, daß** mindestens eines der Nukleotide, insbesondere mehrere der Nukleotide, "Locked Nucleic Acids" ("LNA's") sind oder mindestens eines der Nukleotide, insbesondere alle Nukleotide, Phosphorothioate sind, vorzugsweise daß mehrere der Nukleotide "Locked Nucleic Acids" ("LNA's") sind.

5. Oligonukleotid gemäß Anspruch 4, **dadurch gekennzeichnet, daß** die "LNA's" am 5'- und 3'-Ende des Oligonukleotids liegen, vorzugsweise jeweils die letzten 2-5 Nukleotide, insbesondere jeweils die letzten 3 oder 4 Nukleotide, am 3'- und 5'-Ende des Oligonukleotids "LNA's" sind
und/oder
daß > 6, insbesondere ≥ 8 zusammenhängende Nukleotide im Oligonukleotid keine "LNA's" sind, vorzugsweise daß von den im Sequenzbereich gemäß jeweiligem Unterpunkt (a) abgebildeten Nukleotiden des Oligonukleotids gemäß einer der Abbildungen 1 bis 16 Unterpunkt (b) bis (k) nur jeweils höchstens eines oder keines der Nukleotide eine "LNA" ist.

6. Polynukleotid-Konstrukt enthaltend in zwei voneinander getrennten Bereichen die beiden Nukleotidteilsequenzen Abschn. I und Abschn. III gemäß einem der Unterpunkte (l) - (n) oder die beiden Nukleotidteilsequenzen Helix I und Helix III gemäß einem der Unterpunkte (o) - (q) in einer der Abbildungen 1/27, 3/27, 4/27, 5/27, 6/27, 7/27, 8/27, 9/27, 11/27, 12/27, 13/27 oder 15/27, wobei es sich bei dem Polynukleotid-Konstrukt um ein Ribozym, vorzugsweise ein "hammerhead" Ribozym, oder ein DNA-Enzym, vorzugsweise ein DNA-Enzym vom Typ 10-23 oder 12-32, handelt.

7. Polynukleotid-Konstrukt gemäß Anspruch 6, **dadurch gekennzeichnet, daß** es sich um ein DNA-Enzym enthaltend mindestens die Nukleotidteilsequenzen Abschn. I und Abschn. III gemäß einem der Unterpunkte (l) bis (n), vorzugsweise (n), in einer der Abbildungen 1/27, 3/27, 4/27, 5/27, 6/27, 7/27, 8/27, 9/27, 11/27, 12/27 oder 13/27,
- vorzugsweise 1/27, 3/27, 7/27 oder 11/27, insbesondere 1/27 oder 3/27, oder
- vorzugsweise 4/27, 6/27, 8/27 oder 12/27, insbesondere 4/27 oder 12/27
handelt.

8. Polynukleotid-Konstrukt gemäß einem der Ansprüche 6 bis 7, **dadurch gekennzeichnet, daß** es mindestens eine modifizierte oder unmodifizierte Ribose, mindestens eine modifizierte oder unmodifizierte Phosphodiesterbindung und/oder mindestens eine modifizierte oder unmodifizierte Base aufweist.

9. Oligonukleotid gemäß einem der Ansprüche 1 bis 5 oder Polynukleotid-Konstrukt gemäß einem der Ansprüche 6 bis 8, **dadurch gekennzeichnet, daß** dieses an einen Träger; insbesondere Protein, vorzugsweise tet-, Transportin oder Ferritin; gebunden und/oder in einem Liposom verpackt ist.

10. Zelle enthaltend mindestens ein Oligonukleotid gemäß einem der Ansprüche 1 bis 5 oder 9 und/oder ein Polynukleotid-Konstrukt gemäß einem der Ansprüche 6 bis 8.

11. Arzneimittel enthaltend mindestens ein Oligonukleotid gemäß einem der Ansprüche 1 bis 5 oder 9, ein Polynukleotid-Konstrukt gemäß einem der Ansprüche 6 bis 8 und/oder eine Zelle gemäß Anspruch 10 sowie gegebenenfalls geeignete Hilfs- und/oder Zusatzstoffe.

12. Diagnostikum enthaltend mindestens ein Oligonukleotid gemäß einem der Ansprüche 1 bis 5 oder 9, ein Polynukleotid-Konstrukt gemäß einem der Ansprüche 6 bis 8 und/oder eine Zelle gemäß Anspruch 10 sowie gegebenenfalls geeignete Zusatzstoffe.

13. Verwendung eines Oligonukleotids gemäß einem der Ansprüche 1 bis 5 oder 9, eines Polynukleotid-Konstrukts gemäß einem der Ansprüche 6 bis 8 und/oder einer Zelle gemäß Anspruch 10 zur Herstellung eines Arzneimittels zur Behandlung von Schmerz, insbesondere von chronischem Schmerz, taktiler Allodynie, thermisch ausgelöstem Schmerz, und/oder Entzündungsschmerz.

14. Verwendung eines Oligonukleotids gemäß einem der Ansprüche 1 bis 5 oder 9, eines Polynukleotid-Konstrukts gemäß einem der Ansprüche 6 bis 8 und/oder einer Zelle gemäß Anspruch 9 zur Herstellung eines Arzneimittels zur Behandlung von Harninkontinenz; auch von neurogenen Blasensymptomen; Pruritus, Tumoren, Entzündungen; insbesondere von VR1-Rezeptor-assoziierten Entzündungen mit Symptomen wie Asthma; sowie von allen mit VR1 zusammenhängenden Krankheitssymptomen.

15. Verfahren zur Identifizierung schmerzmodulierender Substanzen, **dadurch gekennzeichnet, daß** die Identifizierung über eine Quantifizierung der Bindung mindestens eines, vorzugsweise markierten, Oligonukleotids gemäß einem der Ansprüche 1 bis 5 oder 9 und/oder eines Polynukleotid-Konstrukts gemäß einem der Ansprüche 6 bis 8 an eine RNA erfolgt.

16. Verfahren zur Identifizierung schmerzmodulierender Substanzen mit folgenden Verfahrensschritten:
(a) gentechnische Manipulation mindestens einer Zelle (Testzelle) mit mindestens einem Oligonukleotid gemäß einem der Ansprüche 1 bis 5 oder 9 und/oder eines Polynukleotid-Konstrukts gemäß einem der Ansprüche 6 bis 8,
(a') parallele gentechnische Manipulation mindestens einer identischen Zelle (Kontrollzelle) entweder
• unterbleibend,
• unter Durchführung der Manipulation parallel ohne Oligonukleotid oder Polynukleotid-Konstrukt oder
• mit einem veränderten, nicht mehr den Ansprüchen 1 bis 5 oder 9 entsprechenden Oligonukleotid oder Polynukleotidkonstrukt,
(b) parallele Inkubation einer zu testenden Substanz unter geeigneten Bedingungen mit mindestens einer Test-Zelle und mindestens einer Konrollzelle und/oder einer Präparation aus solchen Zelle, die mindestens ein Rezeptorprotein, ausgewählt aus der Vanilloid-Rezeptor-Familie, vorzugsweise den VR-1-Rezeptor, synthetisiert hat
(c) Messung der Bindung der Testsubstanz an dem von den Zellen synthetisierten Protein oder Messung mindestens eines der durch die Bindung der Testsubstanz an das Protein veränderten funktionellen Parameter,
(d) Identifizierung der Substanzen über das Ausmaß der Differenz zwischen dem Meßwert bei der Testzelle und dem bei der Kontrollzelle.

17. Verfahren gemäß Anspruch 16, **dadurch gekennzeichnet, daß** die Zelle bereits vor den Verfahrensschritten (a) und (a') gentechnisch manipuliert wird.

18. Verfahren gemäß Anspruch 17, **dadurch gekennzeichnet, daß** die gentechnische Manipulation die Messung mindestens eines der durch die Testsubstanz veränderten funktionellen Parameter erlaubt.

19. Verfahren gemäß einem der Ansprüche 17 oder 18, **dadurch gekennzeichnet, daß** durch die gentechnische Manipulation eine in der Zelle nicht endogen exprimierte Form eines Mitglieds der Vanilloid-Rezeptor-Familie, vorzugsweise der VR-1-Rezeptor, exprimiert oder ein Reportergen eingeführt wird.

20. Verfahren gemäß einem der Ansprüche 16 bis 19, **dadurch gekennzeichnet, daß** die Messung der Bindung über die Verdrängung eines bekannten markierten Liganden eines Mitglieds der Vanilloid-Rezeptor-Familie, vorzugsweise des VR-1-Rezeptors, erfolgt.

21. Verfahren gemäß einem der Ansprüche 16 bis 20, **dadurch gekennzeichnet, daß** zwischen den parallelen Verfahrensschritten (a) und (a') und dem Verfahrensschritt (b) ≥ 8 h, vorzugsweise ≥ 12 h, insbesondere ≥ 24 h, vergehen.

22. Verfahren zur Diagnose von Krankheitsbildern, die mit veränderter Expression von Genen der Vanilloid-Rezeptor-Familie verbunden sind, **dadurch gekennzeichnet, daß** die Diagnose über eine Quantifizierung der Bindung eines Oligonukleotids gemäß einem der Ansprüche 1 bis 5 oder 9 und/oder mindestens einem Polynukleotid-Konstrukt gemäß einem der Ansprüche 6 bis 8 an eine RNA erfolgt.

## Claims

1. An oligonucleotide containing or corresponding to a base sequence according to one of subpoints (b) to (j) in one of Figures 1/27, 2/27, 4/27, 5/27, 6/27, 7/27, 8/27, 9/27, 10/27, 11/27, 12/27, 13/27, 14/27, 15/27 or 16/27 or a sequence differing therefrom by at most one different base, the base difference not being located in the sequence domain shown in subpoint (a), **characterised in that** the oligonucleotide has a length of 15 to 30, preferably of 15 to 25, in particular 17 to 19, or exactly 18, nucleotides.

2. An oligonucleotide containing or corresponding to a base sequence according to subpoint (k) in one of Figures 1/27, 2/27, 4/27, 5/27, 6/27, 7/27, 8/27, 9/27, 10/27, 11/27, 12/27, 13/27, 14/27, 15/27 or 16/27 or a sequence differing therefrom by preferably one and at most two different bases, the base difference(s) not being located in the sequence domain shown in subpoint (a), **characterised in that** the oligonucleotide has a length of 15 to 30, preferably of 15 to 25, in particular 17 to 19, or exactly 18, nucleotides.

3. An oligonucleotide according to either of claim 1 or claim 2, **characterised in that** it comprises at least one modified or unmodified ribose, at least one modified or unmodified phosphodiester bond and/or at least one modified or unmodified base.

4. An oligonucleotide according to any one of claims 1 to 3, **characterised in that** at least one of the nucleotides, in particular two or more of the nucleotides, are "locked nucleic acids" (LNAs) or at least one of the nucleotides, in particular all of the nucleotides, are phosphorothioates, preferably **in that** two or more of the nucleotides are "locked nucleic acids" (LNAs).

5. An oligonucleotide according to claim 4, **characterised in that** the LNAs are located at the 5' and 3' end of the oligonucleotide, preferably in each case the final 2-5 nucleotides, in particular in each case the final 3 or 4 nucleotides, on the 3' and 5' end of the oligonucleotide are LNAs
and/or
**in that** > 6, in particular ≥ 8 contiguous nucleotides in the oligonucleotide are not LNAs, preferably **in that**, of the nucleotides shown in the sequence domain according to the particular subpoint (a) of the oligonucleotide according to one of Figures 1 to 16 subpoints (b) to (k), at most one or none of the nucleotides is in each case an LNA.

6. A polynucleotide construct containing in two separate domains the two nucleotide subsequences frag. I and frag. III according to one of subpoints (l)-(n) or the two nucleotide subsequences helix I and helix III according to one of subpoints (o)-(q) in one of Figures 1/27, 3/27, 4/27, 5/27, 6/27, 7/27, 8/27, 9/27, 11/27, 12/27, 13/27 or 15/27, the polynucleotide construct comprising a ribozyme, preferably a hammerhead ribozyme, or a DNA enzyme, preferably a type 10-23 or 12-32 DNA enzyme.

7. A polynucleotide construct according to claim 6, **characterised in that** it comprises a DNA enzyme containing at least the nucleotide subsequences frag. I and frag. III according to one of subpoints (l)-(n), preferably (n), in one of Figures 1/27, 3/27, 4/27, 5/27, 6/27, 7/27, 8/27, 9/27, 11/27, 12/27 or 13/27,
- preferably 1/27, 3/27, 7/27 or 11/27, in particular 1/27 or 3/27, or
- preferably 4/27, 6/27, 8/27 or 12/27, in particular 4/27 or 12/27.

8. A polynucleotide construct according to any one of claims 6 to 7, **characterised in that** it comprises at least one modified or unmodified ribose, at least one modified or unmodified phosphodiester bond and/or at least one modified or unmodified base.

9. An oligonucleotide according to any one of claims 1 to 5 or a polynucleotide construct according to any one of claims 12 to 19, **characterised in that** it is bound on a support, in particular a protein, preferably tet-, transportin or ferritin, and/or is packaged in a liposome.

10. A cell containing at least one oligonucleotide according to any one of claims 1 to 5 or 9 and/or a polynucleotide construct according to any one of claims 6 to 8.

11. A pharmaceutical preparation containing at least one oligonucleotide according to any one of claims 1 to 5 or 9, a polynucleotide construct according to any one of claims 6 to 8 and/or a cell according to claim 10, optionally together with suitable auxiliary substances and/or additives.

12. A diagnostic aid containing at least one oligonucleotide according to any one of claims 1 to 5 or 9, a polynucleotide construct according to any one of claims 6 to 8 and/or a cell according to claim 10, optionally together with suitable additives.

13. Use of an oligonucleotide according to any one of claims 1 to 5 or 9, a polynucleotide construct according to any one of claims 6 to 8 and/or a cell according to claim 10 for the production of a pharmaceutical preparation for the treatment of pain, in particular chronic pain, tactile allodynia, thermally induced pain and/or inflammatory pain.

14. Use of an oligonucleotide according to any one of claims 1 to 5 or 9, a polynucleotide construct according to any one of claims 6 to 8 and/or a cell according to claim 9 for the production of a pharmaceutical preparation for the treatment of urinary incontinence; also of neurogenic bladder symptoms; pruritus, tumours, inflammation; in particular VR1 receptor-associated inflammation with symptoms such as asthma; together with any disease symptoms associated with VR1.

15. A method for the identification of pain-modulating substances, **characterised in that** identification is made by quantification of the binding of at least one, preferably labelled, oligonucleotide according to any one of claims 1 to 5 or 9 and/or a polynucleotide construct according to any one of claims 6 to 8 onto an RNA.

16. A method for the identification of pain-modulating substances comprising the following method steps:
(a) genetic engineering manipulation of at least one cell (test cell) with at least one oligonucleotide according to any one of claims 1 to 5 or 9 and/or a polynucleotide construct according to any one of claims 6 to 8,
(a') parallel genetic engineering manipulation of at least one identical cell (control cell), which is either
• not carried out,
• carried out with performance of the manipulation in parallel with the oligonucleotide or polynucleotide construct or
• carried out with a modified oligonucleotide or polynucleotide construct which is no longer according to claims 1 to 5 or 9,
(b) parallel incubation of a substance to be tested under suitable conditions with at least one test cell and at least one control cell and/or a preparation made from such a cell, which has synthesised at least one receptor protein selected from the vanilloid receptor family, preferably the VR-1 receptor,
(c) measurement of the binding of the test substance onto the protein synthesised by the cells or measurement of at least one functional parameter modified by binding of the test substance onto the protein,
(d) identification of the substances by the extent of the difference between the measured value for the test cell and that for the control cell.

17. A method according to claim 16, **characterised in that** the cell has already been subjected to genetic engineering manipulation before method steps (a) and (a').

18. A method according to claim 17, **characterised in that** the genetic engineering manipulation permits the measurement of at least one of the functional parameters modified by the test substance.

19. A method according to either of claim 17 or claim 18, **characterised in that** the genetic engineering manipulation results in the expression of a form, not endogenously expressed in the cell, of a member of the vanilloid receptor family, preferably the VR-1 receptor, or in the introduction of a reporter gene.

20. A method according to any one of claims 16 to 19, **characterised in that** binding is measured by the displacement of a known, labelled ligand of a member of the vanilloid receptor family, preferably the VR-1 receptor.

21. A method according to any one of claims 16 to 20, **characterised in that** ≥ 8 h, preferably ≥ 12 h, in particular ≥ 24 h elapse between the parallel method steps (a) and (a') and method step (b).

22. A method for the diagnosis of clinical pictures which are associated with modified expression of genes of the vanilloid receptor family, **characterised in that** the diagnosis is made by means of quantification of the binding of an oligonucleotide according to any one of claims 1 to 5 or 9 and/or at least one polynucleotide construct according to any one of claims 6 to 8 onto an RNA.

## Revendications

1. Oligonucléotide contenant ou correspondant à une séquence de bases selon l'une des subdivisions (b) à (j) dans l'une des formules 1/27, 2/27, 3/27, 4/27, 5/27, 6/27, 7/27, 8/27, 9/27, 10/27, 11/27, 12/27, 13/27, 14/27, 15/27 où 16/27 ou contenant ou correspondant à une séquence s'en distinguant au maximum par une base qui s'en écarte, l'écart de la base ne se trouvant pas dans le domaine de séquence représenté à la subdivision (a), **caractérisé en ce qu'**il présente une longueur de 15 à 30, avantageusement de 15 à 25, en particulier de 17 à 19 ou exactement de 18 nucléotides.

2. Oligonucléotide contenant ou correspondant à une séquence de bases selon la subdivision (k) dans l'une des figures 1/27, 2/27, 3/27, 4/27, 5/27, 6/27, 7/27, 8/27, 9/27, 10/27, 11/27, 12/27, 13/27, 14/27, 15/27 ou 16/27 ou contenant ou correspondant à une séquence qui s'en distingue au maximum de deux bases, avantageusement d'une seule base, qui s'en écartent, l'écart ou les écarts de la base ou des bases ne se trouvant pas dans le domaine de séquence figurant à la subdivision (a), **caractérisé en ce qu'**il présente une longueur de 15 à 30, avantageusement de 15 à 25, en particulier de 17 à 19 ou exactement de 18 nucléotides.

3. Oligonucléotide suivant l'une des revendications 1 ou 2, **caractérisé en ce qu'**il présente au moins un ribose modifié ou non modifié, au moins une liaison phosphodiester modifiée ou non modifiée et/ou au moins une base modifiée ou non modifiée.

4. Oligonucléotide suivant l'une des revendications 1 à 3, **caractérisé en ce qu'**au moins l'un des nucléotides, en particulier plusieurs des nucléotides sont des « Locked Nucleid Acids » (« LNA ») ou bien au moins l'un des nucléotides, en particulier tous les nucléotides, sont des phosphorothioates, avantageusement **en ce que** plusieurs des nucléotides sont des « Locked Nucleid Acids » (« LNA »).

5. Oligonucléotide suivant la revendication 4, **caractérisé en ce que** les « LNA » sont situés aux extrémités 5' et 3' de l'oligonucléotide, de préférence chacun des 2 à 5 derniers nucléotides, en particulier chacun des troisième ou quatrième derniers nucléotides, se trouvant avantageusement aux extrémités 3' et 5' de l'oligonucléotide « LNA »,
et/ou
dans lesquels plus de 6, en particulier 8 ou plus de 8, nucléotides en liaison dans l'oligonucléotide n'étant pas des « LNA », avantageusement dans lesquels parmi les nucléotides, représentés dans le domaine de séquence selon la subdivision (a) respective, de l'oligonucléotide selon l'une des figures 1 à 16, aux subdivisions (b) à (k), au maximum dans chaque cas un seul des nucléotides est un « LNA », ou aucun n'est un « LNA ».

6. Produit d'assemblage polynucléotidique contenant dans deux régions séparées l'une de l'autre les deux séquences nucléotidiques partielles fragment I et fragment III selon l'une des subdivisions (l) - (n) ou bien les deux séquences nucléotidiques partielles hélice I et hélice III selon l'une des subdivisions (o) - (q) dans l'une des figures 1/27, 3/27, 4/27, 5/27, 6/27, 7/27, 8/27, 9/27, 11/27, 12/27, 13/27 ou 15/27, le produit d'assemblage polynucléotidique étant un ribozyme, avantageusement un ribozyme « hammerhead », ou bien un DNA-enzyme, avantageusement un DNA-enzyme du type 10 - 23 ou 12 - 32.

7. Produit d'assemblage polynucléotidique suivant la revendication 6, **caractérisé en ce qu'**il consiste en un DNA-enzyme contenant au moins les séquences partielles nucléotidiques fragment I et fragment III selon l'une des subdivisions (l) à (n), avantageusement (n), dans l'une des figures 1/27, 3/27, 4/27, 5/27, 6/27, 7/27, 8/27, 9/27, 11/27, 12/27 ou 13/27.
- avantageusement 1/27, 3/27, 7/27 ou 11/27, en particulier 1/27 ou 3/27,
ou bien
- avantageusement 4/27, 6/27, 8/27 ou 12/27, en particulier 4/27 ou 12/27.

8. Produit d'assemblage polynucléotidique selon l'une des revendications 6 à 7, **caractérisé en ce qu'**il présente au moins un ribose modifié ou non modifié, au moins une liaison phosphodiester modifiée ou non modifiée et/ou au moins une base modifiée ou non modifiée.

9. Oligonucléotide suivant l'une des revendications 1 à 5 ou produit d'assemblage polynucléotidique selon l'une des revendications 6 à 8, **caractérisé en ce qu'**il est lié à un support, en particulier une protéine, avantageusement tet-, transportine ou ferritine ; et/ou empaqueté dans un liposome.

10. Cellule contenant au moins un oligonucléotide selon l'une des revendications 1 à 5 ou 9 et/ou un produit d'assemblage polynucléotidique selon l'une des revendications 6 à 8.

11. Médicament contenant au moins un oligonucléotide selon l'une des revendications 1 à 5 ou 9, un produit d'assemblage polynucléotidique selon l'une des revendications 6 à 8 et/ou une cellule selon la revendication 10 ainsi que, le cas échéant, des substances auxiliaires et/ou des additifs convenables.

12. Produit diagnostique contenant au moins un oligonucléotide selon l'une des revendications 1 à 5 ou 9, un produit d'assemblage polynucléotidique selon l'une des revendications 6 à 8 et/ou une cellule selon la revendication 10 ainsi que, le cas échéant, des additifs convenables.

13. Utilisation d'un oligonucléotide selon l'une des revendications 1 à 5 ou 9, d'un produit d'assemblage polynucléotidique selon l'une des revendications 6 à 8 et/ou d'une cellule selon la revendication 10 pour la préparation d'un médicament destiné au traitement de la douleur, en particulier d'une douleur chronique, de l'allodynie tactile, d'une douleur déclenchée thermiquement et/ou d'une douleur inflammatoire.

14. Utilisation d'un oligonucléotide selon l'une des revendications 1 à 5 ou 9, d'un produit d'assemblage polynucléotidique selon l'une des revendications 6 à 8 et/ou d'une cellule selon la revendication 9 pour la préparation d'un médicament destiné au traitement de l'incontinence d'urine, ainsi que de symptômes vésicaux d'origine nerveuse ; du prurit, de tumeurs, d'inflammations, en particulier d'inflammations associées au récepteur VR1 avec des symptômes tels que l'asthme, ainsi que de tous les symptômes de maladies en rapport avec le récepteur VR1.

15. Méthode d'identification de substances modulant la douleur, **caractérisée en ce que** l'identification est effectuée au moyen d'une quantification de la liaison d'au moins un oligonucléotide avantageusement marqué selon l'une des revendications 1 à 5 ou 9 et/ou d'un produit d'assemblage polynucléotidique selon l'une des revendications 6 à 8, à un RNA.

16. Méthode d'identification de substances modulant la douleur, comprenant les étapes suivantes:
(a) manipulation par génie génétique d'au moins une cellule (cellule d'essai) avec au moins un oligonucléotide conforme à l'une des revendications 1 à 5 ou 9 et/ou un produit d'assemblage polynucléotidique conforme à l'une des revendications 6 à 8,
(a') manipulation par génie génétique parallèle d'au moins une cellule identique (cellule témoin)
• non effectuée,
• avec conduite de la manipulation en parallèle sans oligonuclélotide ni produit d'assemblage polynucléotidique ou
• avec un oligonucléotide ou produit d'assemblage polynucléotidique modifié, n'étant plus conforme à l'une des revendications 1 à 5 ou 9,
(b) incubation parallèle d'une substance à tester dans des conditions convenables avec au moins une cellule d'essai et au moins une cellule témoin et/ou avec une préparation d'une cellule qui a synthétisé au moins une protéine qui est un récepteur choisi dans la famille des récepteurs vanilloïdes, avantageusement le récepteur VR-1,
(c) mesure de la liaison de la substance testée à la protéine synthétisée par les cellules ou mesure d'au moins un paramètre fonctionnel modifié par la liaison de la substance testée à la protéine,
(d) identification des substances d'après la grandeur de la différence entre la valeur de mesure pour la cellule d'essai et la valeur de mesure pour la cellule témoin.

17. Méthode suivant la revendication 16, **caractérisée en ce que** la cellule est manipulée par génie génétique avant même les étapes (a) et (a') de la méthode.

18. Méthode suivant la revendication 17, **caractérisée en ce que** la manipulation par génie génétique permet la mesure d'au moins l'un des paramètres fonctionnels modifiés par la substance testée.

19. Méthode suivant l'une des revendications 17 ou 18, **caractérisée en ce que** la manipulation par génie génétique exprime une forme, non exprimée de façon endogène dans la cellule, d'un représentant de la famille des récepteurs vanilloïdes, avantageusement le récepteur VR-1, ou bien un gène rapporteur est introduit.

20. Méthode suivant l'une des revendications 16 à 19, **caractérisée en ce que** la mesure de la liaison est effectuée au moyen du déplacement d'un ligand marqué connu d'un représentant de la famille des récepteurs vanilloïdes, avantageusement le récepteur VR-1.

21. Méthode suivant l'une des revendications 16 à 20, **caractérisée en ce que** le temps écoulé entre les étapes parallèles (a) et (a') et l'étape (b) de la méthode est égal ou supérieur à 8 heures, avantageusement égal ou supérieur à 12 heures, en particulier égal ou supérieur à 24 heures.

22. Méthode de diagnostic de signes cliniques qui sont en liaison avec l'expression modifiée de gènes de la famille des récepteurs vanilloïdes, **caractérisée en ce que** le diagnostic est effectué au moyen d'une quantification de la liaison d'un oligonucléotide selon l'une des revendications 1 à 5 ou 9 et/ou d'au moins un produit d'assemblage polynucléotidique selon l'une des revendications 6 à 8 à un RNA.
